(19) Europäisches Patentamt / European Patent Office / Office européen des brevets

(11) **EP 4 100 877 B1**

(12) **EUROPÄISCHE PATENTSCHRIFT**

(45) Veröffentlichungstag und Bekanntmachung des Hinweises auf die Patenterteilung:
**09.08.2023   Patentblatt 2023/32**

(21) Anmeldenummer: **21703245.7**

(22) Anmeldetag: **02.02.2021**

(51) Internationale Patentklassifikation (IPC):
**G06V 10/50** (2022.01)     **G06V 10/44** (2022.01)
**A61B 5/00** (2006.01)      **G06V 10/20** (2022.01)
**A61B 5/107** (2006.01)     **A61B 5/026** (2006.01)

(52) Gemeinsame Patentklassifikation (CPC):
**A61B 5/0071; A61B 5/0261; G06V 10/255;
G06V 10/446; G06V 10/50;** A61B 5/0036;
A61B 5/1076; A61B 5/489; A61B 5/7264;
G06V 2201/031

(86) Internationale Anmeldenummer:
**PCT/EP2021/052449**

(87) Internationale Veröffentlichungsnummer:
**WO 2021/156266 (12.08.2021 Gazette 2021/32)**

(54) **COMPUTERIMPLEMENTIERTES VERFAHREN, COMPUTERPROGRAMM UND OPERATIONSSYSTEM ZUR BESTIMMUNG DES BLUTVOLUMENFLUSSES DURCH EINEN ABSCHNITT EINES BLUTGEFÄSSES IN EINEM OPERATIONSBEREICH**

COMPUTER-IMPLEMENTED METHOD, COMPUTER PROGRAM AND OPERATING SYSTEM FOR DETERMINING BLOOD VOLUME FLOW THROUGH A SECTION OF A BLOOD VESSEL IN AN OPERATING AREA

MÉTHODE, PROGRAMME INFORMATIQUE ET SYSTÈME D'EXPLOITATION MIS EN OEUVRE PAR ORDINATEUR POUR DÉTERMINER LE DÉBIT DU VOLUME SANGUIN À TRAVERS UNE PARTIE D'UN VAISSEAU SANGUIN DANS UNE ZONE D'OPÉRATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **03.02.2020   DE 102020102681**

(43) Veröffentlichungstag der Anmeldung:
**14.12.2022   Patentblatt 2022/50**

(73) Patentinhaber: **Carl Zeiss Meditec AG
07745 Jena (DE)**

(72) Erfinder:
• **NABER, Ady
  73432 Aalen (DE)**
• **NAHM, Werner
  74426 Bühlerzell (DE)**
• **HAUGER, Christoph
  73431 Aalen (DE)**
• **GUCKLER, Roland
  89081 Ulm (DE)**

(74) Vertreter: **Gauss, Nikolai
  Pfiz/Gauss Patentanwälte PartmbB
  Tübingerstraße 26
  70178 Stuttgart (DE)**

(56) Entgegenhaltungen:
EP-A1- 3 047 796      EP-B1- 3 047 796
DE-A1-102010 055 772   DE-B4-102010 055 772
DE-T2- 60 038 730     US-A1- 2007 244 393

**Beschreibung**

Die Erfindung betrifft ein computerimplementiertes Verfahren zur Bestimmung des Blutvolumenflusses durch einen Abschnitt eines Blutgefäßes in einem Operationsbereich anhand eines Fluorophors. Dabei wird eine Mehrzahl von Bildern, die auf Fluoreszenzlicht in Form von Licht mit innerhalb eines Fluoreszenzspektrums des Fluorophors liegenden Wellenlängen basieren und die den Abschnitt des Blutgefäßes zu unterschiedlichen Aufnahmezeitpunkten zeigen, bereitgestellt. Durch Verarbeiten der bereitgestellten Bilder werden ein Durchmesser und eine Länge des Abschnitts des Blutgefäßes sowie ein Zeitintervall zu einer Ausbreitung des Fluorophors durch das Blutgefäß ermittelt, das eine charakteristische Transitzeit für das Fluorophor in dem Blutgefäß beschreibt. Die Erfindung betrifft auch ein Computerprogramm und ein Operationssystem zur Bestimmung des Blutvolumenflusses durch einen Abschnitt eines Blutgefäßes in einem Operationsbereich anhand eines Fluorophors.

Das Bestimmen des Blutvolumenflusses ist z. B. bei neurochirurgischen Operationen von Interesse, da sich damit der Erfolg einer Bypass Revaskularisierung, eines Aneurysmen-Clipping oder einer Angiombehandlung kontrollieren lässt. Die Bewegung der im menschlichen Blut enthaltenen Plasmaproteine kann sichtbar gemacht werden, indem das Blut mit einem Fluoreszenzfarbstoff, z. B. mit dem Farbstoff Indocyaningrün (ICG) versetzt wird, der sich an Plasmaproteine im Blut bindet und der durch Beleuchten mit Licht einer geeigneten Wellenlänge zu Fluoreszenz angeregt werden kann. Wenn ein solcher Fluoreszenzfarbstoff in die Blutbahn eines Patienten zugeführt wird, kann mittels eines Videokamerasystems, das für das Erfassen des Fluoreszenzlichts des Fluoreszenzfarbstoffs ausgelegt ist, durch Auswerten entsprechender Videobilder auf einen Blutvolumenfluss in den Blutgefäßen eines Patienten geschlossen werden.

Ein computerimplementiertes Verfahren der eingangs genannten Art zur Bestimmung des Blutvolumenflusses durch einen Abschnitt eines Blutgefäßes in einem Operationsbereich ist aus "Claudia Weichelt et al. Quantitative fluorescence angiography for neurosurgical interventions, Biomed Tech 2013, Band 58, Nr. 3, S. 269 - 279" bekannt. Dort ist das Bestimmen des Blutvolumenfluss in einem Blutgefäß beschrieben, in das der Farbstoff ICG zugeführt und zu dem mittels einer Videokamera eine Videosequenz aufgezeichnet wird. Hier werden von einem Operateur zur Bestimmung des Blutvolumenflusses ein Start- und ein Endpunkt eines Abschnitts eines interessierenden Blutgefä-ßes ausgewählt. An diesen Punkten wird die Intensität des Fluoreszenzlichts über die Zeit ermittelt, geglättet und dann der zeitliche Versatz zwischen den Intensitätskurven sowie die Länge des Abschnitts des Blutgefäßes bestimmt. Aus dem zeitlichen Versatz und der Länge wird dann eine Blutflussgeschwindigkeit bestimmt, um daraus anhand eines Querschnitts des Blutgefäßes einen Blutvolumenfluss zu berechnen.

Auf S. 274 in dem Abschnitt "Phantom Measurements" ist in "Claudia Weichelt et al. Quantitative fluorescence angiography for neurosurgical interventions, Biomed Tech 2013, Band 58, Nr. 3, S. 269 - 279" angesprochen, dass der in dieser Publikation berechnete Wert des Blutvolumenflusses von dem während der Experimente gemessenen Wert durch einen von dem Durchmesser des Blutgefäßes abhängigen Faktor abweicht. Es wird jedoch keine Vorschrift für die Berechnung dieses Faktors angegeben.

In "Tsukiyama, A.; Murai, Y.; Matano, F.: Shirokane, K.; Morita, A.: Optical effects on the surrounding structure during quantitative analysis using indocyanine green videoangiography: A phantom vessel study, J. Biophotonics, 2018, 11.-ISSN 1864-0648" ist beschrieben, dass die lokale Intensität von Fluoreszenzlicht, das mittels eines Operationsmikroskops aus einem Beobachtungsfenster in einem Operationsbereich mit einem Blutgefäß erfasst wird, nicht nur von dem Abstand des Beobachtungsfensters zu dem Blutgefäß, sondern auch von der Dicke des Blutgefäßes und von der räumlichen Umgebung des Blutgefäßes abhängt. Dort wird darauf hingewiesen, dass diese Abhängigkeit bei einer quantitativen Analyse von Fluoreszenzlicht, um auf den Blutfluss in einem Blutgefäß zu schließen, beachtet werden muss.

"Xu, J., Song, S., Li, Y., and Wang, R.: Complex-based OCT angiography algorithm recovers microvascular information superior to amplitude or phasebased algorithm in phase-stable systems, Physics in medicine and biology, vol. 63, 19. Dec. 2017, 1 - ISSN 1361-6560" gibt das Untersuchen der Retina mittels OCT an, um durch Auswerten von Phase und Amplitude des OCT-Signals dort insbesondere Blutgefäße zu visualisieren.

Aus "Saito et al., Quantitative Blood Flow Assessment by Multiparameter Analysis of Indocyanine Green Video Angiography, World Neurosurgery, 2018, Band 116, S. 187 - 193" ist eine Analyse mehrerer messbarer Größen in den Videodaten bei Zugabe von ICG und Bestrahlung des Operationsbereichs mit Fluoreszenzanregungslicht bekannt. Hier wird der zeitliche Intensitätsverlauf an nur einer Stelle des Blutgefäßes untersucht und durch Vergleich mit einem Experiment der Gradient als bester Indikator für den Blutvolumenfluss gefunden. Eine konkrete Rechenvorschrift für die Bestimmung des Blutvolumenflusses während einer Operation ist jedoch nicht angegeben.

Das Dokument EP3047796 A1 offenbahrt ein computerimplementiertes Verfahren, ein System zur Durchführung des Verfahrens und ein Computerprogramm mit Programmcode zur Durchführung der Verfahrensschritte zur Bestimmung des Blutvolumenflusses durch einen Abschnitt eines Blutgefäßes in einem Operationsbereich anhand eines Fluorophors nach dem Oberbegrift der Ansprüche 1,16 und 15.

Aufgabe der Erfindung ist es, insbesondere in einer chirurgischen Operation ein genaues Bestimmen des Blutvolumenflusses durch einen Abschnitt eines Blutgefäßes eines Patienten zu ermöglichen.

Diese Aufgabe wird durch das in Anspruch 1 angegebene Verfahren zur Bestimmung des Blutvolumenflusses durch einen Abschnitt eines Blutgefäßes, das in Anspruch 15 angegebene Computerprogramm und die in Anspruch 16 angegebene Vorrichtung gelöst. Vorteilhafte Ausführungsformen und Weiterbildungen der Erfindung sind in den abhängigen Ansprüchen angegeben.

Der Begriff Blutvolumenfluss $I_{Bi}$ bezeichnet vorliegend das Volumen V des Blutes, das pro Zeiteinheit t durch den Abschnitt des Blutgefäßes mit Durchmesser D und Länge L fließt:

$$I_{Bi} = \dot{V} = \left(\frac{D}{2}\right)^2 \pi \frac{L}{t} = \left(\frac{D}{2}\right)^2 \pi v$$

mit

$$v := \frac{L}{t}.$$

Das in Anspruch 1 angegebene erfindungsgemäße computerimplementierte Verfahren zur Bestimmung des Blutvolumenflusses durch einen Abschnitt eines Blutgefäßes in einem Operationsbereich anhand eines Fluorophors umfasst die folgenden Verfahrensschritte:

Es wird eine Mehrzahl von Bildern, die auf Fluoreszenzlicht in Form von Licht mit innerhalb eines Fluoreszenzspektrums des Fluorophors liegenden Wellenlängen basieren und die den Abschnitt des Blutgefäßes zu unterschiedlichen aufeinanderfolgenden Aufnahmezeitpunkten zeigen, bereitgestellt. Durch Verarbeiten der bereitgestellten Bilder werden ein Durchmesser und eine Länge des Abschnitts des Blutgefäßes sowie ein Zeitintervall zu einer Ausbreitung des Fluorophors durch den Abschnitt des Blutgefäßes ermittelt, das eine charakteristische Transitzeit für das Fluorophor in dem Abschnitt des Blutgefäßes beschreibt. Es wird ein Blutgefäß-Modell, welches den Abschnitt des Blutgefäßes als einen Strömungskanal mit einer Länge, mit einer Wand mit einer Wandstärke und mit einem freien Querschnitt Q beschreibt, an wenigstens eines der bereitgestellten Bilder mittels Bildverarbeitung angepasst. Es wird ein Fluidströmungs-Modell $M_F^Q$ zu dem angepassten Blutgefäß-Modell bereitgestellt, das eine lokale Strömungsgeschwindigkeit an unterschiedlichen Positionen über den freien Querschnitt Q des Strömungskanals in dem angepassten Blutgefäß-Modell beschreibt.

Weiter wird ein Fluoreszenzlicht-Modell $M_L^Q$ bereitgestellt, das eine räumliche Wahrscheinlichkeitsdichte für die Intensität des remittierten Lichts an unterschiedlichen Positionen über den freien Querschnitt Q des Strömungskanals in dem angepassten Blutgefäß-Modell beschreibt, das von einem mit Fluorophor versetzten, den freien Querschnitt Q des Strömungskanals in dem angepassten Blutgefäß-Modell durchströmenden Fluid bei Bestrahlung mit Fluoreszenzanregungslicht ausgesendet wird. Außerdem wird ein Blutvolumenflusses als ein durch den Strömungskanal in dem angepassten Blutgefäß-Modell geführter Fluidstrom bestimmt, der anhand des bereitgestellten Fluidströmungs-Modells $M_F^Q$ und des bereitgestellten Fluoreszenzlicht-Modells $M_L^Q$ aus der Länge und dem Durchmesser des Abschnitts des Blutgefäßes sowie der charakteristischen Transitzeit für das Fluorophor in dem Abschnitt des Blutgefäßes berechnet wird.

Für den Abschnitt des Blutgefäßes wird ein Blutgefäß-Modell $M_B^Q$ verarbeitet, welches den Abschnitt des Blutgefäßes als einen Strömungskanal mit einer Länge, mit einer Wand mit einer Wandstärke und mit einem freien Querschnitt Q beschreibt, indem wenigstens eines der bereitgestellten Bilder verarbeitet wird. Außerdem wird ein Fluidströmungs-Modell $M_F^Q$ zu dem Blutgefäß-Modell $M_B^Q$ verarbeitet, das eine lokale Strömungsgeschwindigkeit an unterschiedlichen Positionen über den freien Querschnitt Q des Strömungskanals in dem Blutgefäß-Modell $M_B^Q$ beschreibt. Darüber hinaus wird ein Fluoreszenzlicht-Modell $M_L^Q$ angenommen, das eine räumliche Wahrscheinlichkeitsdichte für die Intensität des remittierten Lichts an unterschiedlichen Positionen über den freien Querschnitt Q des Strömungskanals in dem Blutgefäß-Modell $M_B^Q$ beschreibt, das von einem mit Fluorophor versetzten, den freien Querschnitt Q des Strömungskanals in dem Blutgefäß-Modell $M_B^Q$ durchströmenden Fluid bei Bestrahlung mit Fluoreszenzlicht ausgesendet wird. Schließlich wird der Blutvolumenfluss als ein durch den Strömungskanal in dem Blutgefäß-Modell $M_B^Q$ geführter Fluidstrom bestimmt, der anhand des Fluidströmungs-Modells $M_F^Q$ und des Fluoreszenzlicht-Modells $M_L^Q$ aus der Länge und dem Durchmesser des Abschnitts des Blutgefäßes sowie der charakteristischen Transitzeit für das Fluorophor in dem Abschnitt des Blutgefäßes berechnet wird.

Das Fluidströmungs-Modell $M_F^Q$ bezeichnet eine Abbildung des freien Querschnitts Q, insbesondere von Teilbereichen dieses, auf Strömungsgeschwindigkeiten, wobei die Strömungsgeschwindigkeiten eindimensional in Form des Betrags der Strömungsgeschwindigkeit oder in Form eines n-dimensionalen Strömungsgeschwindigkeitsvektors mit Betrag und Richtung angegeben sein können:

$$M_F^Q : Q \rightarrow \mathbb{R}^n, n \in \mathbb{N}.$$

Das Fluoreszenzlicht-Modell $M_L^Q$ bezeichnet eine Abbildung des freien Querschnitts Q, insbesondere von Teilbereichen dieses, auf eine reelle Zahl:

$$M_L^Q : Q \rightarrow \mathbb{R}.$$

Das Treffen der Modellannahmen anhand des Blutgefäß-Modells $M_B^Q$, des Fluidströmungs-Modells $M_F^Q$ sowie des Fluoreszenzlicht-Modells $M_L^Q$ ermöglicht eine Bestimmung des Blutvolumenflusses durch den Abschnitt des Blutgefä-βes aus den während der Operation bereitgestellten Bildern anhand einer konkreten Rechenvorschrift. Dadurch ist das Verfahren in der Praxis während einer Operation anwendbar. Aufgrund der verschiedenen Parameter der Modellannahmen ist das Verfahren zur Bestimmung des Blutvolumenflusses flexibel an verschiedene Szenarien anpassbar. Das Verfahren eignet sich deshalb auch für eine Messung des Volumenflusses eines anderen Mediums als Blut durch ein Gefäß mit einem anderen Schichtaufbau, wobei das Medium eine charakteristische Fluidströmung und das Fluoreszenzlicht charakteristische Eigenschaften aufweisen, welche in den Modellannahmen repräsentiert werden können. Durch die Anpassung der Modellannahmen an die speziellen Gegebenheiten bei Operationen kann auch eine erhöhte Genauigkeit des mit dem Verfahren bestimmten Blutvolumenflusses erzielt werden.

Das erfindungsgemäße Verfahren beruht auf den folgenden Annahmen: Der betrachtete Abschnitt des Blutgefäßes ist in den bereitgestellten Bildern deutlich und vollständig sichtbar und befindet sich in der Fokusebene der Bilderfassungseinrichtung. Die Parameter der Bilderfassungseinrichtung sowie die Parameter des Blutgefäß-Modells $M_B^Q$, des Fluidströmungs-Modells $M_F^Q$ sowie des Fluoreszenzlicht-Modells $M_L^Q$ werden nicht verändert während der Bestimmung des Blutvolumenflusses.

Es ist von Vorteil, wenn das Blutgefäß-Modell $M_B^Q$ ein Hohlzylinder mit der zu dem Abschnitt des Blutgefäßes ermittelten Länge, dem ermittelten Durchmesser und der ermittelten Wandstärke ist. Dies vereinfacht das Blutgefäß-Modell $M_B^Q$ und somit auch die Berechnung des Blutvolumenflusses, wodurch Rechenzeit eingespart werden kann.

Des Weiteren ist es vorteilhaft, wenn das Fluidströmungs-Modell $M_F^Q$ eine laminare Fluidströmung durch den Strömungskanal des Blutgefäß-Modells $M_B^Q$ beschreibt. Diese Maßnahme vereinfacht das Fluidströmungs-Modell $M_F^Q$ und somit auch die Berechnung des Blutvolumenflusses, wodurch ebenfalls Rechenzeit eingespart werden kann.

Zur Bestimmung des Blutvolumenflusses durch den Abschnitt des Blutgefäßes ist es außerdem vorteilhaft, wenn das Fluidströmungs-Modell $M_F^Q$ und das Fluoreszenzlicht-Modell $M_L^Q$ einen lokalen Ausschnitt des Blutgefäß-Modells $M_B^Q$ beschreiben, sodass das Urbild des Fluidströmungs-Modells $M_F^Q$ und des Fluoreszenzlicht-Modells $M_L^Q$ einem Teilbereich des freien Querschnitts Q des Blutgefäß-Modells $M_B^Q$ entspricht. Dadurch wird das Verfahren wiederum vereinfacht und in der Praxis anwendbar. Insbesondere ist es hilfreich, wenn das Fluidströmungs-Modell $M_F^Q$ und das Fluoreszenzlicht-Modell $M_L^Q$ statt auf dem freien Querschnitt Q nur auf dem Durchmesser des freien Querschnitts Q entlang einer orthogonal zur Mittellinie des Blutgefäßes verlaufenden, die Mittellinie schneidenden Linie definiert sind. Jeder Punkt x $\in D \subset Q$ entspricht dann einer Eindringtiefe von Photonen in das Blutgefäß.

Das Fluoreszenzlicht-Modell $M_L^Q$ beruht vorzugsweise auf einer in silico Simulation einer Bestrahlung des Blutgefäß-Modells $M_B^Q$ mit Fluoreszenzanregungslicht, bei der Photonen als an Streuzentren gestreute Teilchen angenommen werden. Dabei weisen die Streuzentren im Strömungskanal und in der Wand des Blutgefäß-Modells $M_B^Q$ eine jeweils charakteristische Streuzentrenverteilung auf. Das Blutgefäß-Modell $M_B^Q$ wird dabei als Schicht-Modell mit drei Schichten dargestellt: Blutgefäßwand - Strömungskanal - Blutgefäßwand. Die Bewegung einer Vielzahl von Photonen wird in diesem Schicht-Modell anhand zuvor festgelegter Parameter für die Absorption, Streuung und Streu-Anisotropie der Photonen in dem Medium innerhalb der Schichten und an Schichtgrenzen simuliert wie in der Publikation "L. Wang, S. Jacques, Monte Carlo Modeling of Light Transport in Multi-layered Tissues in Standard C, Computer Methods and Programs in Biomedicine, Band 47, Nr. 2, S. 131 - 146, 1995" beschrieben, auf die hiermit vollumfänglich Bezug genommen und deren Offenbarung in die Beschreibung dieser Erfindung einbezogen wird. Zur Bestimmung des Fluoreszenzlicht-Modells $M_L^Q$ anhand der in silico Simulation ist es dabei von Vorteil, wenn das Urbild des Fluoreszenzlicht-Modells $M_L^Q$ einer Sehne des freien Querschnitts Q des Blutgefäß-Modells $M_B^Q$ entspricht, welche die Eindringtiefe der Photonen in das Blutgefäß-Modell $M_B^Q$ bei Bestrahlung mit Fluoreszenzanregungslicht darstellt. Eine Sehne eines Querschnitts Q bezeichnet dabei eine Strecke zwischen zwei beliebigen Punkten auf dem Rand des Querschnitts Q, sodass die Strecke innerhalb des Querschnitts Q verläuft. Das Fluoreszenzlicht-Modell $M_L^Q$ bildet dann die Eindringtiefe x auf den Anteil der aus dem Blutgefäß-Modell $M_B^Q$ remittierten Photonen ab, deren maximale Eindringtiefe in dem Blutgefäß-Modell $M_B^Q$ während der Simulation dem Wert x entspricht. Das Fluoreszenzlicht-Modell $M_L^Q(x)$ kann dabei als Wahrscheinlichkeitsdichte aufgefasst werden mit

$$\int_Q M_L^Q(x)\, dx = 1.$$

Eine vorteilhafte Ausführungsform des Verfahrens sieht vor, dass das Fluidströmungs-Modell $M_F^Q$ einem relativen Fluidströmungs-Modell $M_{Fr}^Q$ entspricht, das ein relatives Strömungsprofil in Form einer relativen Strömungsgeschwindigkeit an unterschiedlichen Positionen über den freien Querschnitt Q des Strömungskanals in dem Blutgefäß-Modell $M_B^Q$ in Relation zu einer Referenz-Strömungsgeschwindigkeit $v_R$ beschreibt. Das relative Fluidströmungs-Modell $M_{Fr}^Q$ ergibt sich aus dem Fluidströmungsmodell $M_F^Q$ wie folgt:

$$M_{Fr}^Q(\mathrm{x}) := \frac{M_F^Q(\mathrm{x})}{v_R}, x \in Q.$$

Das Verwenden eines relativen Fluidströmungs-Modells $M_{Fr}^Q$ hat den Vorteil, dass die Strömungsgeschwindigkeiten an verschiedenen Positionen im Verhältnis zueinander und zu der Referenz-Strömungsgeschwindigkeit $v_R$ bekannt sind. Somit kann aus der Beobachtung einer Geschwindigkeit an einer Stelle in dem Blutgefäß auf die Referenz-Strömungsgeschwindigkeit $v_R$ geschlossen werden. Die Referenz-Strömungsgeschwindigkeit $v_R$ wird dabei vom Anwender ausgewählt. Dabei kann die Referenz-Strömungsgeschwindigkeit $v_R$ z. B.

- als eine Strömungsgeschwindigkeit $v_x$ des Fluidströmungs-Modells an einer bestimmten Stelle $x \in Q$ in dem Blutgefäß-Modell $M_B^Q$ mit

$$v_x = M_F^Q(x) \text{ für ein } x \in Q,$$

- als eine maximale Strömungsgeschwindigkeit $v_{max}$ des Fluidströmungs-Modells in dem Blutgefäß-Modell $M_B^Q$ mit

$$v_{max} = max\{M_F^Q(x) | x \in Q\},$$

- als eine mittlere Strömungsgeschwindigkeit $v_{Mittel}$ des Fluidströmungs-Modells in dem Blutgefäß-Modell $MB$ mit

$$v_{Mittel} = \frac{\int_Q M_F^Q(x) dx}{\int_Q dx}$$

gewählt werden.

Indem das Fluidströmungs-Modell $M_F^Q$ ein relatives Fluidströmungs-Modell $M_{Fr}^Q$ ist, das eine lokale relative Strömungsgeschwindigkeit an unterschiedlichen Positionen über den freien Querschnitt Q des Strömungskanals in dem Blutgefäß-Modell $M_B^Q$ in Relation zu einer Referenz-Strömungsgeschwindigkeit $v_R$ beschreibt, lässt sich die Bestimmung des Blutvolumenflusses $I_{BI}$ durch einen Abschnitt eines Blutgefäßes in einem Operationsbereich anhand eines Fluorophors mit einer erhöhten Genauigkeit erreichen.

Diese Maßnahme ermöglicht es nämlich, den in der oben genannten Publikation von Claudia Weichelt et al. angesprochenen, aus einem Experiment ermittelten Fehler bei der Berechnung des Blutvolumenstromes in Form eines Faktors für den dort ermittelten Blutvolumenfluss aus dem relativen Fluidströmungs-Modell $M_{Fr}^Q$ und dem Fluoreszenzlicht-Modell $M_L^Q$ zu bestimmen.

Um die Genauigkeit des Verfahrens zur Blutvolumenflussbestimmung zu verbessern, schlägt die Erfindung vor, den in der oben genannten Publikation von Claudia Weichelt et al. gemessenen Faktor analytisch zu bestimmen. In einer vorteilhaften Weiterbildung der Erfindung ist deshalb vorgesehen, die beobachtete Strömungsgeschwindigkeit $v_{beobachtet}$ anhand eines Korrekturfaktors wie folgt zu korrigieren:

$$v_{korrigiert} := v_{beobachtet} \cdot k\_v_R,$$

wobei der Korrekturfaktor $k\_v_R$ in Abhängigkeit von der Referenz-Strömungsgeschwindigkeit $v_R$ des relativen Fluidströmungs-Modells $M_{Fr}^Q$ ermittelt wird.

Der Erfindung liegt dabei die Erkenntnis zugrunde, dass die dem ermittelten Zeitintervall entsprechende charakteristische Transitzeit τ für das Fluorophor in dem Abschnitt eines Blutgefäßes mit dem Durchmesser D und der Länge L, die einer beobachteten Strömungsgeschwindigkeit

$$v_{beobachtet} := \frac{L}{\tau}$$

entspricht, insbesondere von der Eindringtiefe des Fluoreszenzlichts in das Blutgefäß abhängt, weil sich der Anteil des remittierten Fluoreszenzlichts mit der Eindringtiefe des Fluoreszenzlichts in das Blutgefäß ändert. Das Fluoreszenzlicht-Modell $M_L^Q$ beeinflusst daher die in dem Abschnitt des Blutgefäßes beobachtete Strömungsgeschwindigkeit $v_{beobachtet}$.

Durch eine Kombination des Fluidströmungs-Modells $M_F^Q$ mit dem Fluoreszenzlicht-Modell $M_L^Q$ kann das Wissen über das aus verschiedenen Eindringtiefen remittierte Fluoreszenzlicht in die Berechnung der Strömungsgeschwindigkeit in dem Blutgefäß-Modell $M_B^Q$ mit einbezogen werden.

Die Erfinder haben nämlich erkannt, dass die Intensität des remittierten Lichts keiner Gleichverteilung über alle Eindringtiefen entspricht, sondern dass vielmehr der Anteil des remittierten Lichts aus bestimmten Eindringtiefen besonders hoch und aus anderen Eindringtiefen sehr gering ist. Dieser Zusammenhang wird durch das Fluoreszenzlicht-Modells $M_L^Q$ beschrieben.

Eine Erkenntnis der Erfindung ist es deshalb insbesondere, dass sich der Korrekturfaktor aus dem Fluidströmungs-Modell $M_F^Q$ und dem Fluoreszenzlicht-Modell $M_L^Q$, die beide auf dem Blutgefäß-Modell $M_B^Q$ basieren, ermitteln lässt.

Dazu wird die in dem Blutgefäß-Modell $M_B^Q$ beobachtete Strömungsgeschwindigkeit $v_{Modell}$ als ein Erwartungswert über die beobachteten lokalen Strömungsgeschwindigkeiten des Fluidströmungs-Modells $M_F^Q$ in Abhängigkeit von der in dem Fluoreszenzlicht-Modell $M_L^Q$ angegebenen räumlichen Wahrscheinlichkeitsdichte für die Intensität des remittierten Lichts an unterschiedlichen Positionen über den freien Querschnitt Q des Strömungskanals 94 in dem Blutgefäß-Modell ($M_B^Q$) wie folgt berechnet:

$$v_{Modell} = \int_Q M_F^Q(x) M_L^Q(x) dx.$$

Entspricht das Fluidströmungs-Modell $M_F^Q$ einem relativen Fluidströmungs-Modell $M_{Fr}^Q$ zu einer gewählten Referenz-Strömungsgeschwindigkeit $v_R$, so gilt:

$$v_{Modell} = \int_Q \left( M_{Fr}^Q(x)\, v_R \right) M_L^Q(x) dx$$

und somit

$$v_R = v_{Modell} \frac{1}{\int_Q M_{Fr}^Q(x)\, M_L^Q(x) dx}.$$

Aus der in dem Blutgefäß-Modell $M_B^Q$ beobachteten Strömungsgeschwindigkeit $v_{Modell}$ kann also mittels des Korrekturfaktors

$$k\_v_R := \frac{1}{\int_Q \; M_{Fr}^Q(x) \; M_L^Q(x)dx}$$

auf die Referenz-Strömungsgeschwindigkeit $v_R$ geschlossen werden. Dieser Korrekturfaktor hängt von der ausgewählten Referenz-Strömungsgeschwindigkeit $v_R$ in dem Fluidströmungsmodell $M_F^Q$ ab.

Unter der Annahme, dass die tatsächlich in dem Abschnitt des Blutgefäßes beobachtete Strömungsgeschwindigkeit $v_{beobachtet}$ der in dem Blutgefäß-Modell $M_B^Q$ beobachteten Strömungsgeschwindigkeit $v_{Modell}$ entspricht, d. h.

$$v_{Modell} = v_{beobachtet} \, ,$$

kann aus der in dem Abschnitt des Blutgefäßes beobachteten Strömungsgeschwindigkeit $v_{beobachtet}$ die in dem Abschnitt des Blutgefäßes zu erwartende Referenz-Strömungsgeschwindigkeit $\overline{v_R}$ ermittelt werden. Die in dem Abschnitt des Blutgefäßes beobachtete Strömungsgeschwindigkeit $v_{beobachtet}$ wird also anhand des Korrekturfaktors $k\_v_R$ bei Wahl einer geeigneten Referenz-Strömungsgeschwindigkeit $v_R$ korrigiert:

$$v_{korrigiert} := \overline{v_R} = v_{beobachtet} \cdot k_{v_R}.$$

Um z. B. aus der in dem Abschnitt des Blutgefäßes beobachteten Strömungsgeschwindigkeit $v_{beobachtet}$ die mittlere Strömungsgeschwindigkeit in dem Abschnitt des Blutgefäßes über alle Positionen des freien Querschnitts Q des Strömungskanals des Blutgefäß-Modells $M_B^Q$ zu erhalten, kann als Referenz-Strömungsgeschwindigkeit $v_{R\_Mittel}$

die mittlere Strömungsgeschwindigkeit $v_{Mittel}$ in dem Fluidströmungs-Modell $M_F^Q$ wie folgt berechnet werden:

$$v_{Mittel} := \frac{\int_Q \; M_F^Q(x)dx}{\int_Q \; dx}.$$

Aus der Referenz-Strömungsgeschwindigkeit $v_{R\_Mittel}$ lässt sich das zugehörige relative Fluidströmungs-Modell $M_{Fr\_Mittel}^Q$ wie folgt berechnen:

$$M_{Fr\_Mittel}^Q := \frac{M_F^D}{v_{Mittel}}.$$

Aus dem relativen Fluidströmungs-Modell $M_{Fr\_Mittel}^Q$ und dem Fluoreszenzlicht-Modell $M_L^Q$ kann dann ein Korrekturfaktor $k_{Mittel}$ wie folgt bestimmt werden:

$$k_{Mittel} := \frac{1}{\int_Q \; M_{Fr\_Mittel}^Q(x) \; M_L^Q(x)dx}.$$

Der Korrekturfaktor $k_{Mittel}$ ermöglicht es also, die in dem Abschnitt des Blutgefäßes beobachtete Strömungsgeschwindigkeit $v_{beobachtet}$ wie folgt auf einen in dem Fluidströmungsmodell $M_F^Q$ zu erwartenden Wert für die mittlere Strömungsgeschwindigkeit $\overline{v_{Mittel}}$ in dem Blutgefäß korrigieren:

$$v_{korrigiert} = \overline{v_{Mittel}} = v_{beobachtet} \cdot k_{Mittel}.$$

In dem Verfahren wird also der durch den Strömungskanal in dem Blutgefäß-Modell $M_B^Q$ geführte Fluidstrom berechnet, indem aus dem relativen Fluidströmungs-Modell $M_{Fr}^Q$ zu der Referenz-Strömungsgeschwindigkeit $v_R$ sowie dem Fluoreszenzlicht-Modell $M_L^Q$ ein Korrekturfaktor $k\_v_R$ bestimmt wird und indem aus der Länge L des Abschnitts $90_1, 90_2, 90_3, ...$ des Blutgefäßes 88 sowie aus der charakteristischen Transitzeit $\tau$ für das Fluorophor in dem Abschnitt $90_1, 90_2, 90_3, ...$ des Blutgefäßes 88 eine Fluorophor-Ausbreitungsgeschwindigkeit bestimmt wird, die mittels des Korrekturfaktors $k\_v_R$ auf einen der Referenz-Strömungsgeschwindigkeit $v_R$ entsprechenden Wert korrigiert wird. Der Korrekturfaktor $k\_v_R$ wird dabei als Inverse des Erwartungswerts der relativen Strömungsgeschwindigkeiten in dem relativen Fluidströmungs-Modell $M_{Fr}^Q$ in Abhängigkeit von der durch das Fluoreszenzlicht-Modell $M_L^Q$ beschriebenen räumlichen Wahrscheinlichkeitsdichte für die Intensität des remittierten Lichts an unterschiedlichen Positionen über den freien Querschnitt Q des Strömungskanals in dem Blutgefäß-Modell $M_B^Q$ nach der folgenden Vorschrift bestimmt:

$$k\_v_R = \frac{1}{\int_Q M_{Fr}^Q(x) \, M_L^Q(x) dx}.$$

Vorzugsweise erfolgt die Bestimmung des Blutvolumenflusses anhand eines Look-up-Tables, der vorberechnete Korrekturfaktoren für verschiedene Fluidströmungs-Modelle $M_F^Q$ und/oder verschiedene Fluoreszenzlicht-Modelle $M_L^Q$ enthält. Der zugehörige Korrekturfaktor kann beispielsweise in Abhängigkeit von dem Durchmesser D des Hohlzylinders des Blutgefäß-Modells $M_B^Q$ vorberechnet und als ein Tupel [Durchmesser, k] in einer Tabelle abgespeichert werden. Dadurch wird Rechenzeit eingespart und das Verfahren vereinfacht.

Zur Bestimmung der Länge, des mittleren Durchmessers, einer Mittellinie des Abschnitts des Blutgefäßes als Parameter des Blutgefäß-Modells $M_B^Q$, zur Bestimmung der Parameter des Fluidströmungs-Modells $M_F^Q$ und/oder der Parameter des Fluoreszenzlicht-Modells $M_L^Q$ ist es von Vorteil, ein ausgewähltes Bild aus den bereitgestellten Bildern anhand eines Kriteriums in Bezug auf die Bildhelligkeit der einzelnen Bildpunkte des Bildes, d. h. Intensität der Bildpunkte, als ein Maß für die Intensität des mittels der Bilderfassungseinrichtung erfassten remittierten Lichts zu ermitteln.

Dieses Kriterium entspricht einem Zustand, bei dem das Blutgefäß in dem Bild mit dem Fluoreszenzmittel maximal angefüllt ist. Diese Maßnahme erhöht die Genauigkeit der Bestimmung des Blutvolumenflusses.

Zur Bestimmung der Länge des Abschnitts des Blutgefäßes und/oder des Durchmessers des Abschnitts des Blutgefäßes wird vorzugsweise eine Mittellinie des Abschnitts des Blutgefäßes in wenigstens einem der bereitgestellten Bilder bestimmt. Die Mittellinie bildet eine Mittelachse des Abschnitts des Blutgefäßes, sodass der Abstand zur Blutgefäß-Wand an jeder Stelle der Mittellinie gleich ist. Die Mittellinie kann mittels Bildverarbeitung aus einem bereitgestellten Bild ermittelt werden. Dadurch wird eine automatische Bestimmung der Mittellinie gewährleistet, sodass möglichst geringe oder gar keine Interaktion des Operateurs während des Verfahrens zur Bestimmung des Blutvolumenflusses notwendig ist, sodass das Verfahren in der Praxis anwendbar ist.

Dafür wird in einem ausgewählten Bild zuerst anhand eines Bildsegmentierungsverfahrens der Abschnitt des Blutgefäßes ermittelt. Eine Segmentierung bezeichnet dabei ein Bild, das für jeden Pixel eine Klasse angibt, zu welcher der Pixel gehört. Insbesondere handelt es sich bei der Segmentierung um ein Binärbild, in dem der Wert 1 bedeutet, dass der Pixel zum Abschnitt des Blutgefäßes gehört, und der Wert 0, dass der Pixel nicht zum Abschnitt des Blutgefäßes gehört. Um eine kurze Rechenzeit zu gewährleisten, eignen sich als Segmentierungsverfahren insbesondere adaptive Thresholding Verfahren, wie z. B. in der Publikation "Nobuyuki Otsu, A threshold selection method from gray-level histograms, IEEE Trans. Sys. Man. Cyber. 1979, Band 9, Nr. 1, S. 62-66" beschrieben, auf die hiermit vollumfänglich Bezug genommen und deren Offenbarung in die Beschreibung dieser Erfindung einbezogen wird.

Es können anstelle dieser Verfahren auch andere Segmentierungsverfahren verwendet werden, die einem Fachmann aus der Literatur bekannt sind, insbesondere Verfahren zur Segmentierung von Blutgefäßen in medizinischen Bildern. Der Operateur kann die Segmentierung anpassen und/oder den Abschnitt des Blutgefäßes auswählen, in dem der Blutvolumenfluss vermessen werden soll. Die Verwendung eines Bildsegmentierungsverfahrens hat den Vorteil, dass das Verfahren möglichst automatisch ohne Aufwand für den Operateur ablaufen kann und es sich somit für die Verwendung während einer Operation in der Praxis eignet.

Zur Bestimmung der Mittellinie aus der Segmentierung des Abschnitts des Blutgefäßes werden vorzugsweise in einem ersten Schritt Pixel auf der Mittelachse des Abschnitts des Blutgefäßes durch Verarbeiten der bereitgestellten Bilder, insbesondere der Segmentierung, ermittelt. Dafür können morphologische Operationen wie die sogenannte Erosion oder sogenannte Voronoi-Diagramme oder auch andere Algorithmen auf die segmentierten Bilder angewendet werden, z. B. ein Skeletonization Algorithmus wie beschrieben in dem Artikel "Fixed Topology Skeletons, P. Golland, W. Grimson, International Conference on Computer Vision and Pattern Recognition (CVPR), 2000", auf den hiermit vollumfänglich Bezug genommen und dessen Offenbarung in die Beschreibung dieser Erfindung einbezogen wird.

Aus den einzelnen Pixeln auf der Mittelachse wird dann durch Verbinden benachbarter Pixel ein Polygonzug bestimmt. Um die Genauigkeit des Verfahrens zu erhöhen, wird die Länge des Polygonzugs durch das Anpassen von Verbindungsstrukturen der Pixel entlang der Mittelachse auf Basis ihrer Pixelnachbarschaften minimiert. Dadurch werden Diskretisierungsfehler verringert. Insbesondere wird dabei zu jedem Pixel entlang der Mittellinie die ihn umgebende Pixelnachbarschaft, beispielsweise die 8er-Pixelnachbarschaft, betrachtet. L-förmige Verbindungsstrukturen zwischen drei aufeinander folgenden Pixeln werden dabei durch diagonale Verbindungs-Strukturen ersetzt, um eine Mittellinie mit einer Länge, die möglichst der Länge des Abschnitts des Blutgefäßes entspricht, zu erhalten. Durch das Fitten kontinuierlicher Funktionen, insbesondere Bezier Splines, an den längen-minimierten Polygonzug können Diskretisierungsfehler weiter verringert und damit die Genauigkeit des Verfahrens erhöht werden.

Vorzugweise wird die Transitzeit aus dem Versatz einer Zeitentwicklung der Bildhelligkeit an wenigstens zwei unterschiedlichen Ausschnitten des Abschnitts des Blutgefäßes durch Verarbeiten der bereitgestellten Bilder ermittelt, wobei an die Zeitentwicklung der Bildhelligkeit an den unterschiedlichen Ausschnitten des Abschnitts des Blutgefäßes jeweils eine kontinuierliche Funktion gefittet wird. Die Transitzeit beschreibt ein für das Fluorophor charakteristisches Zeitintervall für die Ausbreitung des Fluorophors in dem Abschnitt des Blutgefäßes. Um die Transitzeit zu ermitteln, werden ein Anfangspunkt und ein Endpunkt auf der Mittellinie des Abschnitts des Blutgefäßes bestimmt, zwischen denen der Blutvolumenfluss ermittelt werden soll.

Der Anfangspunkt liegt dabei in einem Bereich zwischen 5% und 15%, vorzugsweise 10%, der gesamten Länge des Abschnitts des Blutgefäßes und der Endpunkt in einem Bereich zwischen 80% und 95%, vorzugsweise 90%, der gesamten Länge des Abschnitts des Blutgefäßes. Dadurch werden Ungenauigkeiten bei der Bestimmung der Mittellinie, z. B. durch die Erosion der Segmentierung, die besonders am Anfang und am Ende eines Abschnitts eines Blutgefäßes auftreten, vermieden und somit die Genauigkeit des Verfahrens erhöht. Der Anfangspunkt und der Endpunkt können dabei automatisch anhand der Mittellinie und den angegebenen Bereichen bestimmt werden, oder sie können von einem Operateur in dem ausgewählten Bild festgelegt werden. Zu dem Anfangspunkt und dem Endpunkt wird jeweils ein Ausschnitt bestimmt, der den jeweiligen Punkt umgibt, z. B. eine 5x5-Nachbarschaft von Pixeln mit dem Startpunkt oder dem Endpunkt als Mittelpunkt. Um den Intensitätsverlauf, d. h. den Verlauf der Bildhelligkeit an dem jeweiligen Punkt zu ermitteln, wird in wenigstens einer Mehrzahl der bereitgestellten Bilder die Intensität in dem Ausschnitt durch Mittelung der Intensitäten über alle Pixel des Ausschnitts berechnet. Die Mittelung der Intensitäten über den gesamten Ausschnitt erhöht die Genauigkeit des Verfahrens. Die Intensitätswerte am Anfangspunkt und am Endpunkt werden über die verschiedenen Aufnahmezeitpunkte der Bilder abgetragen. An diese Messwerte wird eine kontinuierliche Funktion gefittet, um eine kontinuierliche Zeitentwicklung der Bildhelligkeit an dem jeweiligen Punkt zu erhalten. Insbesondere wird hierfür eine Gamma-Funktion verwendet. Der zeitliche Versatz der Bildhelligkeits-Kurven am Anfangspunkt und am Endpunkt des Abschnitts des Blutgefäßes stellt die Transitzeit dar.

Der Durchmesser des Strömungskanals lässt sich nicht direkt aus der Segmentierung des Abschnitts des Blutgefäßes ermitteln, da die Segmentierung nicht zwischen Strömungskanal und Blutgefäßwand unterscheidet. Nimmt man einen kreisrunden Querschnitt des Blutgefäß-Modells $MB$ an, so lässt sich der Gesamtdurchmesser des Querschnitts des Blutgefäß-Modells $MB$ anhand der Segmentierung bestimmen. Dafür wird ausgehend von Punkten auf der Mittellinie jeweils ein Kreis um den jeweiligen Punkt bestimmt und dessen Radius so lange vergrößert, bis der Kreisrand mit dem Rand der Segmentierung übereinstimmt. Der Mittelwert der Durchmesser der Kreise entspricht dann dem Durchmesser des kreisrunden Querschnitts des Blutgefäß-Modells $M_B^Q$. Um auch den Durchmesser des Strömungskanals zu bestimmen, ist es erforderlich, in dem segmentierten Abschnitt in den bereitgestellten Bildern zwischen Strömungskanal und Blutgefäßwand zu unterscheiden. Um diese Unterscheidung mit möglichst großer Genauigkeit vornehmen zu können, ist es von Vorteil, wenn die Breite des Strömungskanals sowie die Wandstärke des Blutgefäß-Modells $M_B^Q$ anhand eines Kriteriums in Bezug auf den Intensitätsverlauf orthogonal zur Mittellinie des Abschnitts des Blutgefäßes in einem oder mehreren der bereitgestellten Bilder ermittelt werden. Insbesondere ist es dabei von Vorteil, dass das Kriterium in Bezug auf die Kurve des Intensitätsverlaufs_die Krümmung der Kurve des Intensitätsverlaufs orthogonal zu einer Mittellinie des Abschnitts des Blutgefäßes berücksichtig. Eine Erkenntnis der Erfindung ist es nämlich, dass Punkte auf der Grenze zwischen Strömungskanal und Blutgefäßwand jeweils einem Minimum der Krümmung des Intensitätsverlaufs orthogonal zur Mittellinie des Abschnitts des Blutgefäßes entsprechen. Anhand dieses Kriteriums kann dann aus der Segmentierung des Abschnitts des Blutgefäßes eine Segmentierung des Strömungskanals bestimmt werden. Der Durchmesser des Strömungskanals kann dann wie oben für den Gesamtdurchmesser beschrieben durch eine Vergrößerung des Durchmessers von Kreisen auf der Mittellinie des Abschnitts des Blutgefäßes bis zur Übereinstimmung des Kreisrands mit dem Strömungskanalrand bestimmt werden.

Aus der Transitzeit $\tau$, der Länge L und dem Durchmesser D des Strömungskanals des Abschnitts des Blutgefäßes sowie dem Korrekturfaktor $k\_v_R$ lässt sich dann der Blutvolumenfluss berechnen als

$$I_{Bi} = \left(\frac{D}{2}\right)^2 \pi \frac{L}{\tau} \, k\_v_R.$$

Schließlich ist es auch von Vorteil, wenn zu dem berechneten Blutvolumenfluss in dem Abschnitt des Blutgefäßes ein Vertrauensintervall auf Basis des Durchmessers und/oder der Länge des Abschnitts des Blutgefäßes und/oder der Transitzeit und/oder eines Korrekturfaktors und/oder des Blutgefäß-Modells $M_B^Q$ und/oder des Fluidströmungs-Modells $M_F^Q$ und/oder des Fluoreszenzlicht-Modells $M_L^Q$ und/oder der Form einer Mittellinie des Abschnitts des Blutgefäßes anhand von Fehler-Simulationen bestimmt wird. Dadurch wird die Genauigkeit des mit dem Verfahren bestimmten Blutvolumenflusses für den Operateur quantifiziert. Auch diese Maßnahme trägt dazu bei, dass das Verfahren in der Praxis eingesetzt werden kann.

Eine vorteilhafte Weiterbildung des Verfahrens sieht vor, dass zur Bestimmung des Blutvolumenflusses durch ein Blutgefäß in einem Operationsbereich anhand eines Fluorophors das Blutgefäß in mehrere Abschnitte zerlegt und der Blutvolumenfluss ($I_{Bi}$) in den Abschnitten anhand eines vorstehend angegebenen Verfahrens zur Bestimmung des Blutvolumenflusses in einem Abschnitt eines Blutgefäßes bestimmt wird. Dabei wird der Blutvolumenfluss mit der Maßgabe bestimmt, dass an einer Verzweigung des Blutgefäßes die Summe der Blutvolumenflüsse ($I_{Bi}$) zu der Verzweigung der Summe der Blutvolumenflüsse ($I_{Bi}$) von der Verzweigung entspricht. Diesem Verfahren liegt die Annahme zugrunde, dass das Volumen des Blutes über den Verlauf des Blutgefäßes erhalten bleibt. Um dies zu gewährleisten, kann die Bestimmung des Blutvolumenflusses in den einzelnen Abschnitten des Blutgefäßes als ein Optimierungsproblem über alle Abschnitte formuliert werden, bei dem die Volumenerhaltung an Verzweigungen als Nebenbedingung eingeht. Dies hat den Vorteil, dass der Blutvolumenfluss durch die gleichzeitige Bestimmung in mehreren voneinander abhängigen Abschnitten des Blutgefäßes mit einer höheren Genauigkeit ermittelt werden kann.

Die Erfindung erstreckt sich auch auf ein Computerprogramm mit Programmcode zur Durchführung der vorstehend beschriebenen Verfahrensschritte, wenn das Computerprogramm in eine Rechnereinheit geladen und/oder in einer Rechnereinheit durchgeführt wird.

Darüber hinaus erstreckt sich die Erfindung auf ein Operationssystem zur Bestimmung des Blutvolumenflusses ($I_{Bi}$) durch einen Abschnitt eines Blutgefä-βes in einem Operationsbereich anhand eines Fluorophors. Das Operationssystem enthält eine Beleuchtungseinrichtung zur Bereitstellung von Fluoreszenzanregungslicht für den Operationsbereich, eine Bilderfassungseinrichtung zur Bereitstellung einer Mehrzahl von Bildern, die auf Licht mit innerhalb eines Fluoreszenzspektrums des Fluorophors liegenden Wellenlängen basieren und die den Abschnitt des Blutgefäßes zu unterschiedlichen Aufnahmezeitpunkten zeigen, sowie eine Rechnereinheit mit einem Computerprogramm mit Programmcode zur Durchführung der vorstehend beschriebenen Verfahrensschritte zur Bestimmung des Blutvolumenflusses.

Nachfolgend werden vorteilhafte Ausführungsbeispiele der Erfindung beschrieben, die in den Zeichnungen schematisch dargestellt sind.

Es zeigen:

| | |
|---|---|
| Fig. 1 | ein Operationsmikroskop mit einem System zur Bestimmung des Blutvolumenflusses durch einen Abschnitt eines Blutgefäßes in einem Operationsbereich; |
| Fig. 2 | mehrere Bilder des Operationsbereichs mit einem Blutgefäß; |
| Fig. 3 | ein Blutgefäß-Modell; |
| Fig. 4A | eine Segmentierung eines Abschnitts eines Blutgefäßes; |
| Fig. 4B | einen horizontalen Querschnitt eines Blutgefäß-Modells mit Parametern des Blutgefäß-Modells; |
| Fig. 5A bis Fig. 5F | eine Bestimmung einer Mittellinie eines Abschnitts eines Blutgefäßes; |
| Fig. 6A | einen Ausschnitt eines in der Fig. 5E gezeigten Polygonzugs; |
| Fig. 6B | eine Nachverarbeitung des in der Fig. 6A gezeigten Ausschnitts des Polygonzugs; |
| Fig. 7A | ein Blutgefäß; |
| Fig. 7B | einen Abschnitt des Blutgefäßes; |
| Fig. 7C | einen Intensitätsverlauf orthogonal zur Mittellinie eines Abschnitts des Blutgefäßes; |
| Fig. 7D | Strömungskanal-Randpunkte eines Abschnitts eines Blutgefäßes; |
| Fig. 8 | ein Fluidströmungs-Modell mit einem absoluten Strömungsprofil; |
| Fig. 9A | ein relatives Fluidströmungs-Modell; |
| Fig. 9B | ein relatives Fluidströmungs-Modell mit einem relativen Strömungsprofil in Form einer Parabel zu einem Blutgefäß mit einem Durchmesser von 3 mm; |
| Fig. 10 | eine Simulation für die Propagation von Photonen in einem Blutgefäß-Modell zur Ermittlung eines Fluoreszenzlicht-Modells; |
| Fig. 11 | ein anhand einer Simulation ermitteltes Fluoreszenzlicht-Modell; |
| Fig. 12 | ein Ablaufdiagramm eines Simulations-Algorithmus für die Propagation von Photonen in einem Blutgefäß-Modell; |
| Fig. 13A | einen horizontalen Querschnitt eines Abschnitts eines Blutgefäßes mit einem ersten Ausschnitt, der einen Anfangspunkt enthält, und einem zweiten Ausschnitt, der einen Endpunkt enthält; |
| Fig. 13B | einen vertikalen Querschnitt des Abschnitts des Blutgefäßes in der Fig. 13A entlang der Eindringtiefe x mit dem zugehörigen Strömungsprofil; |
| Fig. 13C | ein relatives Fluidströmungs-Modell; |
| Fig. 13D | ein Fluoreszenzlicht-Modell; |
| Fig. 14A | einen horizontalen Querschnitt eines Abschnitts eines Blutgefäßes mit einem ersten Ausschnitt, der einen Anfangspunkt enthält, und einem zweiten Ausschnitt, der einen Endpunkt enthält; |
| Fig. 14B | eine Zeitentwicklung der Intensität in einem ersten Ausschnitt und in einem zweiten Ausschnitt eines Abschnitts eines Blutgefäßes zur Bestimmung einer Transitzeit; |
| Fig. 15 | ein Ausführungsbeispiel mit Verfahrensschritten zur Bestimmung eines Blutvolumenflusses in einem Ausschnitt eines Blutgefäßes in einem Operationsbereich; und |
| Fig. 16 | ein Ausführungsbeispiel mit Verfahrensschritten zur Bestimmung eines Blutvolumenflusses in einem Ausschnitt eines Blutgefäßes in einem Operationsbereich. |

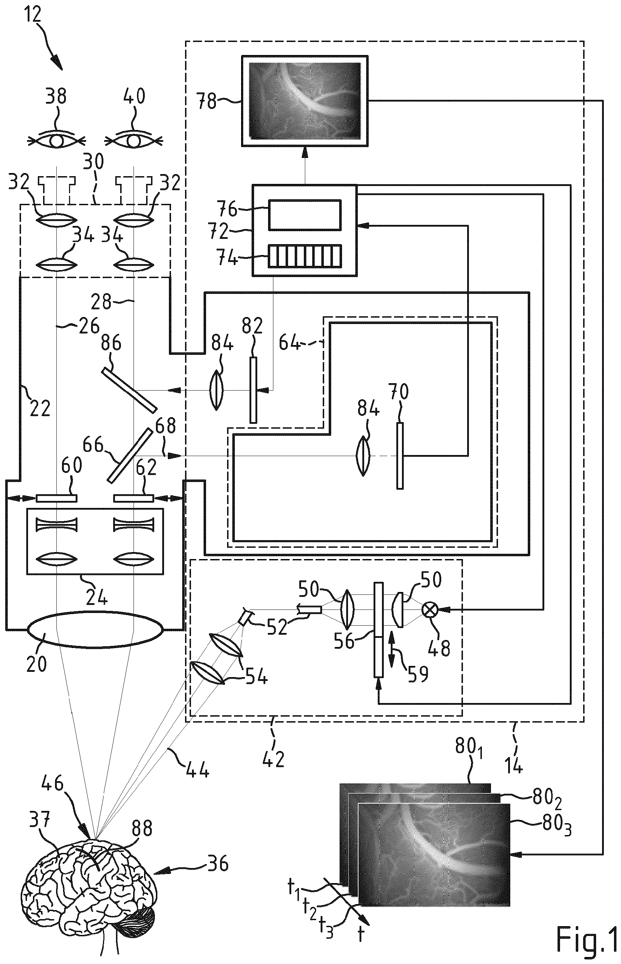

Das in der Fig. 1 gezeigte Operationsmikroskop 12 enthält ein System 14 zur Bestimmung des Blutvolumenflusses $I_{Bi}$ durch einen einzelnen Abschnitt eines Blutgefäßes 88 in einem Operationsbereich 36 und ist für neurochirurgische Operationen ausgelegt. Das Operationsmikroskop 12 hat ein Mikroskop-Hauptobjektiv 20. Das Mikroskop-Hauptobjektiv 20 ist in einem Mikroskop-Grundkörper 22 aufgenommen. Der Mikroskop-Grundkörper 22 enthält ein einstellbares Vergrößerungssystem 24. Das Mikroskop-Hauptobjektiv 20 ist mit einem linken und einem rechten Beobachtungsstrahlengang 26, 28 durchsetzt. An einem Mikroskop-Grundkörper 22 ist ein Binokulartubus 30 angeschlossen. Der Binokulartubus 30 enthält in dem linken und rechten Beobachtungsstrahlengang 26, 28 eine Okularlinse 32 und eine Tubuslinse 34. Über den Binokulartubus 30 kann eine Beobachtungsperson vorliegend einen Operationsbereich 36 an einem Gehirn 37 eines Patienten mit einem linken und rechten Beobachterauge 38, 40 stereoskopisch betrachten.

In dem System 14 zur Bestimmung des Blutvolumenflusses $I_{Bi}$ gibt es eine Beleuchtungseinrichtung 42. Die Beleuchtungseinrichtung 42 stellt mit einem Beleuchtungsstrahlengang 44 Beleuchtungslicht 46 für den Operationsbereich 36 bereit. Die Beleuchtungseinrichtung 42 weist eine Xenon-Lichtquelle 48 auf. Die Beleuchtungseinrichtung 42 enthält weitere optische Elemente in Form von Linsen 50, eines Lichtleiters 52 und eines Beleuchtungsobjektivs 54. Das Licht der Xenon-Lichtquelle 48 wird über ein Linsensystem mit Linsen 50 in einen Lichtleiter 52 eingekoppelt. Aus dem Lichtleiter 52 gelangt Beleuchtungslicht 46 durch ein Beleuchtungsobjektiv 54 zu dem Operationsbereich 36.

Für das Einstellen der spektralen Zusammensetzung des Beleuchtungslichts 46 enthält die Beleuchtungseinrichtung 42 eine schaltbare Filterbaugruppe. Diese Filterbaugruppe enthält ein Beleuchtungsfilter 56. Das Beleuchtungsfilter 56 kann entsprechend dem Pfeil 59 in den Beleuchtungsstrahlengang 44 hinein und aus dem Beleuchtungsstrahlengang 44 heraus bewegt werden.

Das Beleuchtungsfilter 56 ist ein Bandpassfilter. Es ist für Licht aus der Xenon-Lichtquelle 48 im Spektralbereich zwischen 780 nm bis 810 nm durchlässig. Licht im Spektralbereich unterhalb von 810 nm und oberhalb von 780 nm wird dagegen mit dem Beleuchtungsfilter 56 ausgefiltert bzw. stark unterdrückt.

In dem Mikroskop-Grundkörper 22 befindet sich auf der dem Mikroskop-Hauptobjektiv 20 abgewandten Seite des Vergrößerungssystems 24 ein Beobachtungsfilter 60 für den linken Beobachtungsstrahlengang 26 und ein Beobachtungsfilter 62 für den rechten Beobachtungsstrahlengang 28. Die Beobachtungsfilter 60, 62 können entsprechend der Doppelpfeile in den bzw. aus dem Beobachtungsstrahlengang 26, 28 bewegt werden. Das Beleuchtungsfilter 56 einerseits und die Beobachtungsfilter 60, 62 andererseits haben eine Filtercharakteristik, die aufeinander abgestimmt ist. Für das Beobachten des Operationsbereichs 36 mit Fluoreszenzlicht werden das Beleuchtungsfilter 56 in den Beleuchtungsstrahlengang 44 geschaltet und die Beobachtungsfilter 60, 62 in den Beobachtungsstrahlengängen 26, 28 angeordnet.

Das System 14 zur Bestimmung des Blutvolumenflusses $I_{Bi}$ in dem Operationsmikroskop 12 weist eine Bilderfassungseinrichtung 64 auf, die für das Erfassen von Bildern $80_1$, $80_2$, $80_3$, $80_4$, des Operationsbereichs 36 dient. Die Bilderfassungseinrichtung 64 kann Beobachtungslicht von dem Operationsbereich 36 durch das Beobachtungsfilter 62 über einen Auskopplungs-Strahlteiler 66 aus dem rechten Beobachtungsstrahlengang 28 zugeführt werden, der eine optische Achse 68 hat. In der Bilderfassungseinrichtung 64 gibt es einen Bildsensor 70. Der Bildsensor 70 ist empfindlich für die in dem Spektralbereich 810 nm bis 830 nm liegende Emissionswellenlänge des Fluorophors ICG, das einem Patienten für die Bestimmung des Blutvolumenflusses $I_{Bi}$ in einem Blutgefäß in die Blutbahn zugeführt wird.

Der Bildsensor 70 der Bilderfassungseinrichtung 64 ist mit einer Rechnereinheit 72 verbunden. Die Rechnereinheit 72 hat eine Eingabeeinheit 74 und enthält einen Programmspeicher 76. Die Rechnereinheit 72 ist an einen Bildschirm 78 angeschlossen. Auf dem Bildschirm werden zu unterschiedlichen Aufnahmezeitpunkten $t_1$, $t_2$, $t_3$, $t_4$, .... erfasste Bilder $80_1$, $80_2$, $80_3$, $80_4$, ... des Operationsbereichs 36 angezeigt. Die Rechnereinheit 72 steuert ein Display 82. Die Anzeige des Displays 82 wird dem Beobachtungslicht in dem rechten Beobachtungsstrahlengang 28 über eine Linse 84 durch einen Strahlteiler 86 überlagert. Für eine Beobachtungsperson ist damit die Anzeige des Displays 82 gleichzeitig mit dem Operationsbereichs 36 im rechten Okulareinblick des Binokulartubus 30 sichtbar.

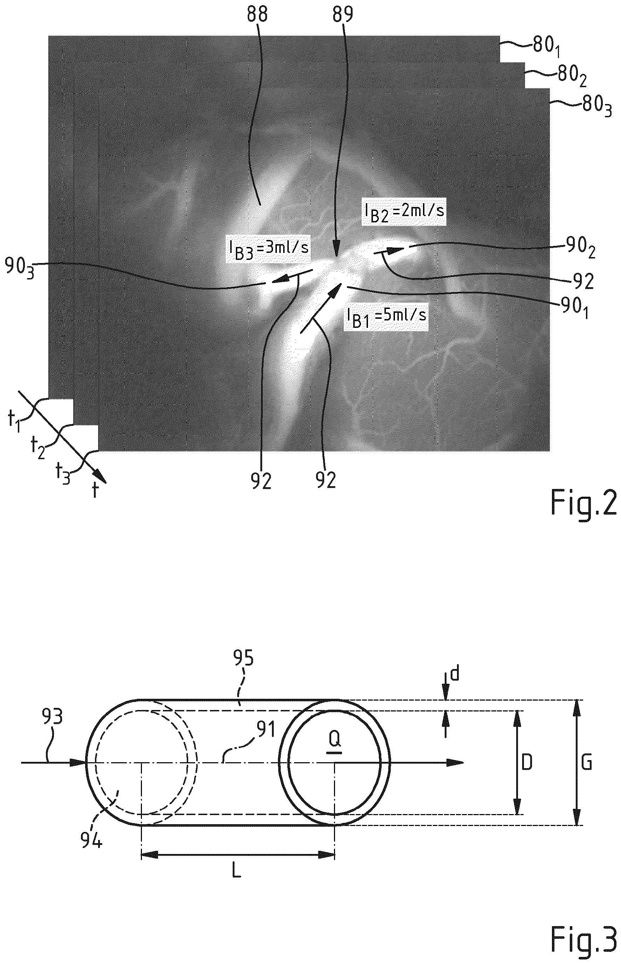

Die Fig. 2 zeigt mehrere Bilder $80_1$, $80_2$, $80_3$, ... des Operationsbereichs 36, die zu unterschiedlichen Aufnahmezeitpunkten $t_1$, $t_2$, $t_3$, .... mittels der Bilderfassungseinrichtung 64 erfasst wurden. Die Bilder $80_1$, $80_2$, $80_3$, ... enthalten jeweils ein Blutgefäß 88, das drei räumlich voneinander getrennte Abschnitte 90, i = 1, 2, 3 hat. In den Bildern $80_1$, $80_2$, $80_3$, ... ist mittels der Pfeile 92 ein Blutvolumenfluss $I_{Bi}$ kenntlich gemacht. Dabei entspricht die Richtung der Pfeile 92 der Richtung des Blutvolumenflusses $I_{Bi}$ und die Länge der Pfeile 92 dem Betrag des Blutvolumenflusses $I_{Bi}$ in den jeweiligen Abschnitten $90_i$ des Blutgefäßes 88.

Der Blutvolumenfluss $I_B$ in dem gesamten Blutgefäß 88 setzt sich aus den Blutvolumenflüssen $I_{Bl}$ in einzelnen Abschnitten $90_i$, i = 1, 2, 3 des Blutgefäßes 88 zusammen. An der in Fig. 2 ersichtlichen Verzweigung 89, die das Blutgefäß 88 aufweist, beträgt der Blutvolumenfluss $I_{B1}$ des zu der Verzweigung 89 strömenden Bluts $I_{B1}$ = 5 ml/s. Der Blutvolumenfluss $I_{B2}$ und $I_{B3}$ in den Abschnitten $90_2$, $90_3$ nach der Verzweigung 89 betragen $I_{B2}$ = 2 ml/s und $I_{B3}$ = 3 ml/s. Es gilt also $I_{B1} = I_{B2} + I_{B3}$, d. h. die Summe der Blutvolumenflüsse $I_{B2}$, $I_{B3}$ nach der Verzweigung 89 entspricht dem Blutvolumenfluss $I_{B1}$ vor der Verzweigung 89. Diese Beziehung besagt, dass das Blutvolumen an einer Verzweigung 89 in dem Blutgefäßsystem erhalten bleibt.

Die Bedingung, dass das Blutvolumen an einer Verzweigung 89 in dem Blutgefäßsystem erhalten bleibt, kann als eine lineare Nebenbedingung für ein Optimierungsproblem herangezogen werden, das über alle Abschnitte $90_i$, i = 1, 2, 3, ... eines Blutgefäßes 88 gleichzeitig den Blutvolumenfluss $I_{Bl}$ anhand der jeweiligen zu dem Abschnitt $90_i$ gegebenen Daten bestimmt.

In den Programmspeicher 76 der Rechnereinheit 72 ist ein Computerprogramm geladen, das zur Bestimmung des Blutvolumenflusses $I_{Bi}$ durch einen Abschnitt $90_i$ eines Blutgefäßes 88 in dem Operationsbereich 36 anhand eines Fluorophors dient.

Das Computerprogramm enthält ein Blutgefäß-Modell $M_B^Q$, das die Geometrie eines Abschnitts $90_i$ des Blutgefäßes 88 beschreibt.

Die Fig. 3 zeigt ein Blutgefäß-Modell $M_B^Q$, das einen Abschnitt $90_i$, i = 1, 2, 3 des in der Fig. 2 gezeigten Blutgefäßes 88, durch welchen das Blut eines Patienten strömt, als einen Hohlzylinder der Länge L und der Zylinderachse 91 beschreibt, der eine Wand 95 mit Wandstärke d hat und der einen Strömungskanal 94 bildet, der durch die Wand 95 des Hohlzylinders begrenzt ist, wobei der Strömungskanal 94 einen kreisrunden Querschnitt Q, einen Innendurchmesser D sowie einen Außendurchmesser G hat und in der Richtung der Pfeile 93 von einer Fluidströmung durchsetzt werden kann.

Die Parameter des Blutgefäß-Modells *MB* werden in dem Computerprogramm, das in den Programmspeicher 76 der Rechnereinheit 72 geladen ist, durch Verarbeiten wenigstens eines der bereitgestellten Bilder $80_1$, $80_2$, $80_3$, $80_4$, ... des Abschnitts $90_i$ des Blutgefäßes 88 ermittelt. Aus der Mehrzahl der Bilder $80_1$, $80_2$, $80_3$, $80_4$, ... wird dazu ein ausgewähltes Bild anhand eines Kriteriums in Bezug auf die Bildhelligkeit der Bildpunkte des Bildes, d. h. der Intensität der Bildpunkte bestimmt. Da das Fluoreszenzlicht eine besonders hohe Intensität von Bildpunkten in den Bildern hervorruft, wird der Zustand, bei dem das Blutgefäß in dem Bild mit dem Fluoreszenzmittel maximal angefüllt ist, durch das folgende Kriterium bestimmt:

$$I_{max} := \max\{I(x) \mid x \in \Omega\}$$

$$A := I_{max} \cdot |\{x \in \Omega \mid \mathrm{I}(x) = I_{max}\}|,$$

wobei $\Omega$ die Menge der Bildpunkte x in einem Bild und I(x) die vorliegend als die Intensität des Bildpunkts x bezeichnete Bildhelligkeit des Bilds an diesem Bildpunkt ist.

Durch die Maximierung des Wertes A wird das Bild ermittelt, das eine hohe maximale Intensität $I_{max}$ und gleichzeitig eine große Anzahl an Pixeln aufweist, die diesen maximalen Intensitätswert annehmen.

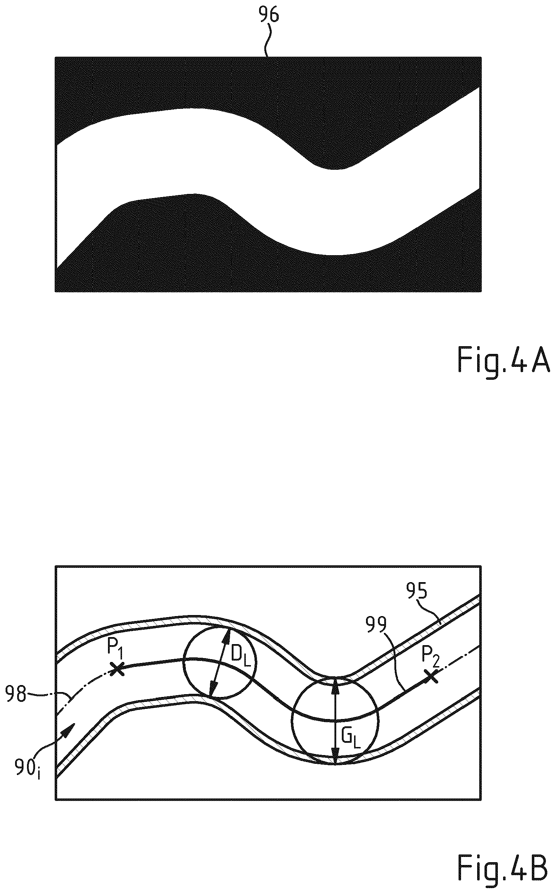

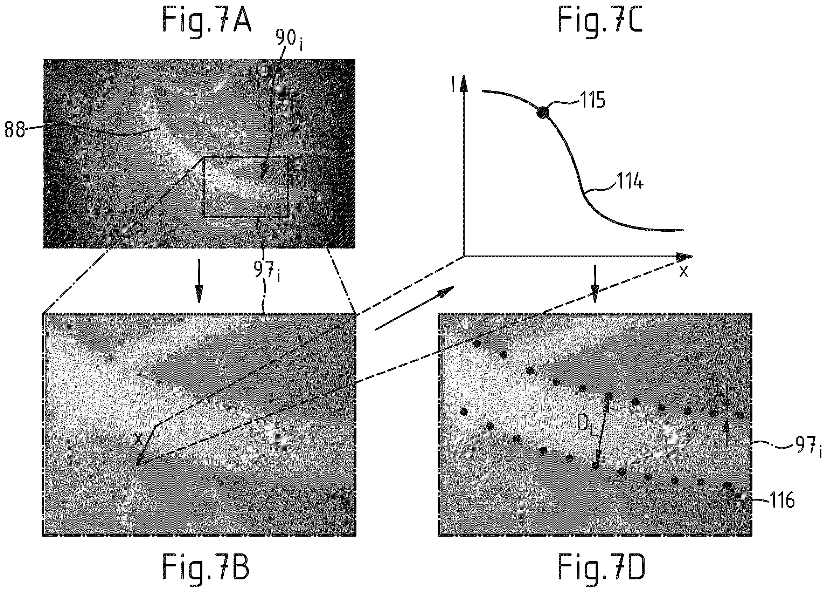

Zu dem Abschnitt $90_i$ des Blutgefäßes 88 wird dann, wie in der Fig. 4A gezeigt, eine Segmentierung 96 sowie ein Anfangspunkt $P_1$ und ein Endpunkt $P_2$ des Abschnitts $90_i$ des Blutgefäßes 88 ermittelt. Die Segmentierung 96 wird mittels Otsu-Thresholding bestimmt, wie in der Publikation "A threshold selection method from gray-level histograms, Nobuyuki Otsu, IEEE Trans. Sys. Man. Cyber. Band 9, Nr. 1, S. 882-886, 1979" beschrieben, auf die hiermit vollumfänglich Bezug genommen und deren Offenbarung in die Beschreibung dieser Erfindung einbezogen wird. Diese initiale Segmentierung 96 wird mittels eines anhand von Fig. 7A bis 7F erläuterten Gradienten-basierten Segmentierungsverfahrens verbessert.

Die Fig. 4B zeigt zu der in der Fig. 4A dargestellten Segmentierung eines Abschnitts $90_i$ des Blutgefäßes 88 einen horizontalen Querschnitt eines Blutgefäß-Modells $M_B^Q$ mit dessen Parametern, die in dem Computerprogramm zur Bestimmung des Blutvolumenflusses $I_{Bi}$ durch den Abschnitt $90_i$ des Blutgefäßes 88 in dem Operationsbereich 36 anhand eines Fluorophors aus der Segmentierung 96 ermittelt werden. Die Mittellinie 98 wird hier mittels Erosion aus der Segmentierung 96 des Abschnitts $90_i$ des Blutgefäßes 88 ermittelt. Es werden dabei ein Anfangspunkt $P_1$ und ein Endpunkt $P_2$ des Abschnitts $90_i$ des Blutgefäßes 88 definiert, zwischen denen der Blutvolumenfluss $I_{Bi}$ bestimmt wird. Der Anfangspunkt $P_1$ und der Endpunkt $P_2$ liegen dabei auf der Mittellinie 98. Der Anfangspunkt $P_1$ liegt außerdem in einem Bereich zwischen 5% und 15%, vorzugsweise 10%, der gesamten Ausdehnung des Abschnitts $90_i$ des Blutgefäßes 88 in die Länge und der Endpunkt $P_2$ in einem Bereich zwischen 80% und 95%, vorzugsweise 90%, der gesamten Ausdehnung des Abschnitts $90_i$ des Blutgefäßes in die Länge. Dadurch werden Ungenauigkeiten bei der Bestimmung der Mittellinie 98, die besonders am Anfang und am Ende eines Abschnitts $90_i$ eines Blutgefäßes 88 auftreten, vermieden.

Der Anfangspunkt $P_1$ und der Endpunkt $P_2$ können automatisch anhand der Mittellinie 98 und den angegebenen Bereichen mittels Bildverarbeitung bestimmt werden, oder sie können von einem Operateur in dem ausgewählten Bild festgelegt werden. Die Länge L des zur Blutvolumenflussbestimmung betrachteten Abschnitts $90_i$ des Blutgefäßes 88 wird bestimmt, indem die Länge des Mittellinienabschnitts 99 der Mittellinie 98 zwischen dem Anfangspunkt $P_1$ und dem Endpunkt $P_2$ ermittelt wird. Zur Bestimmung eines Gesamtdurchmessers G des Abschnitts $90_i$ des Blutgefäßes 88 wird an jedem Punkt entlang der Mittellinie 98 zwischen dem Anfangspunkt $P_1$ und dem Endpunkt $P_2$ ein lokaler Gesamtdurchmesser $G_L$ ermittelt, indem um jeden Punkt ein Kreis definiert und dessen Radius so lange vergrößert wird, bis der Rand des Kreises den Rand des Blutgefäßes 88 berührt. Der Rand des Blutgefäßes 88 kann dabei wie in der Fig. 4A durch das Ermitteln des Rands der Segmentierung 96 des Blutgefäßes 88 bestimmt werden. Der Gesamtdurchmesser G des Blutgefäßes 88 entspricht dann dem Mittelwert aller lokalen Gesamtdurchmesser $G_L$.

Zur Bestimmung eines Durchmessers D des Strömungskanals 94 des Abschnitts 90 des Blutgefäßes 88 wird an jedem Punkt entlang der Mittellinie 98 zwischen dem Anfangspunkt $P_1$ und dem Endpunkt $P_2$ ein lokaler Durchmesser $D_L$ ermittelt, indem um jeden Punkt ein Kreis definiert und dessen Radius so lange vergrößert wird, bis der Rand des Kreises die Innenseite der Wand 95 des Blutgefäßes 88 berührt. Die Innenseite der Wand 95 des Blutgefäßes 88 kann durch das Ermitteln des Rands der Segmentierung 96 des Strömungskanals wie in Fig. 7A bis 7D bestimmt werden. Der Durchmesser D des Strömungskanals 94 entspricht dann dem Mittelwert aller lokalen Durchmesser $D_L$.

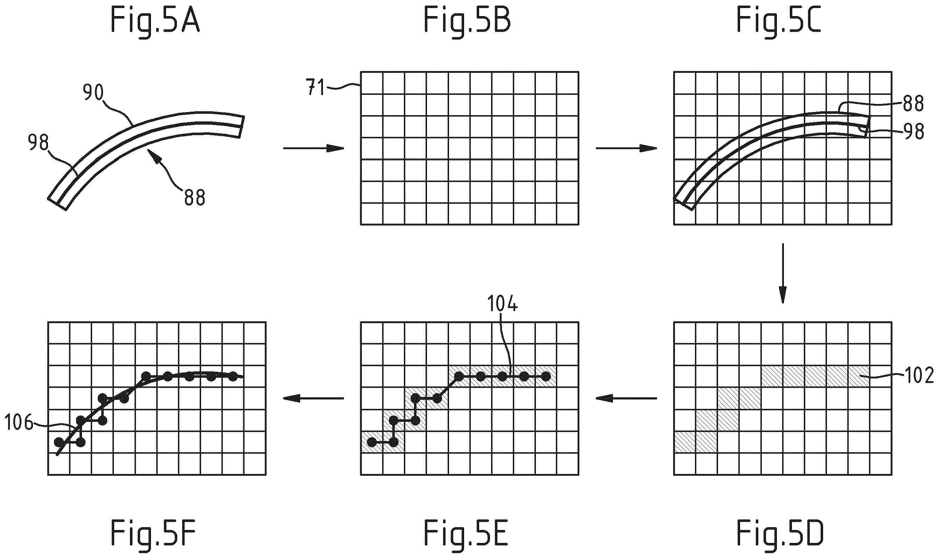

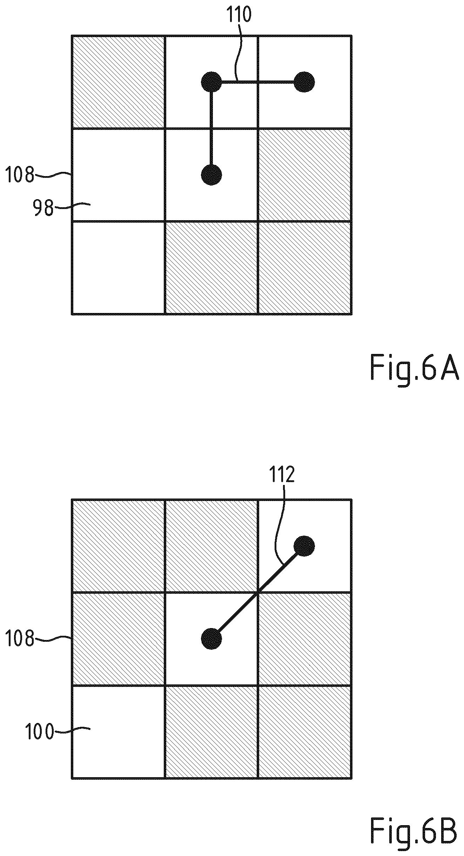

Die Fig. 5A bis 5F erläutern eine Bestimmung einer Mittellinie 98 des Abschnitts 90 des Blutgefäßes 88 sowie eine Bestimmung der Länge L des zur Blutvolumenflussbestimmung betrachteten Abschnitts 90 des Blutgefäßes 88 zwischen dem Anfangspunkt $P_1$ und dem Endpunkt $P_2$ in dem Computerprogramm. Die Fig. 5A zeigt einen Abschnitt 90 des Blutgefäßes 88 mit der Mittellinie 98. In der Bilderfassungseinrichtung 64 wird der Abschnitt 90 des Blutgefäßes 88 auf einen in der Fig. 5B gezeigten Bereich 71 des Bildsensors 70 der Bilderfassungseinrichtung 64 mit einer in der Fig. 5C ersichtlichen Auflösung abgebildet. Die Fig. 5D zeigt die zu der diskretisierten Mittellinie 102 auf dem Bildsensor 70 gehörenden Pixel und die Fig. 5E den Polygonzug 104 der diskretisierten Mittellinie 102. Das Computerprogramm enthält eine Nachverarbeitungsroutine, die diesen Polygonzug 104, wie in der Fig. 6 zu sehen, nachverarbeitet, um bei der Längenbestimmung der Mittellinie 98 zwischen dem Anfangspunkt $P_1$ und dem Endpunkt $P_2$ Diskretisierungsfehler möglichst zu vermeiden. Diese Diskretisierungsfehler betragen, wie in dem Artikel "A. Naber, D. Berwanger, W. Nahm, In Silico Modling of Blood Vessel Segmentations for Estimation of Discretization Error in Spatial Measurement and its Impact on Quantitative Fluorescence Angiography, 41st Annual International Conference of the IEEE Engineering in Medicine and Biology Society (EMBC), 2019" als das Ergebnis von Experimenten beschrieben, im Mittel 6.3%. An den nachverarbeiteten Polygonzug 104 werden, wie in der Fig. 5F gezeigt, kontinuierliche Funktionen 106 in Form von Splines gefittet und die Länge L des Mittellinienabschnitts 99 des Abschnitts 90 des Blutgefäßes 88 zwischen dem Anfangspunkt $P_1$ und dem Endpunkt $P_2$ mittels eines Bogenintegrals berechnet, um den Diskretisierungsfehler weiter zu verringern.

Wie in der Fig. 6A und 6B gezeigt, korrigiert die Nachverarbeitungsroutine des Computerprogramms die Mittellinie 98 des in der Fig. 5E gezeigten Polygonzugs 104 der Mittellinie 98 durch Betrachtung von Pixelnachbarschaften zur Verringerung von Diskretisierungsfehlern. Dafür wird zu jedem Pixel entlang der Mittellinie 98 die ihn umgebende Pixelnachbarschaft 108, hier die 8er-Pixelnachbarschaft 108, betrachtet. L-förmige Verbindungsstrukturen 110 wie in der Fig. 6A werden dabei durch diagonale Verbindungsstrukturen 112 in der Fig. 6B ersetzt. Auf diese Weise wird die Länge der Mittellinie 98 minimiert und es entsteht eine nachverarbeitete Mittellinie 100. Alternativ zu der 8er-Nachbarschaft können auch andere Pixelnachbarschaften betrachtet werden.

Die Fig. 7A bis Fig. 7D erläutern die Bestimmung des Durchmessers D des Strömungskanals 94 sowie der Wandstärke d des Blutgefäß-Modells $M_B^Q$ in dem Computerprogramm anhand eines der mittels der Bilderfassungseinrichtung 64 erfassten und damit bereitgestellten Bilder $80_1$, $80_2$, $80_3$, $80_4$, .... Da eine Wand 95 des Blutgefäßes ein Fluoreszenzsignal streut, ist die Grenze zwischen Strömungskanal 94 und der Wand 95 des Blutgefäßes in einem mittels der Bilderfassungseinrichtung 64 erfassten Bild nicht eindeutig zu erkennen. Die Fig. 7A zeigt ein Blutgefäß 88 mit einem darin ausgewählten Abschnitt $97_i$ eines der Bilder $80_1$, $80_2$, $80_3$, $80_4$, .... In der Fig. 7B ist der ausgewählte Abschnitt $91_i$ des Blutgefäßes 88 zu sehen. Ein örtlicher Intensitätsverlauf I(x) in dem ausgewählten Bild ist als Kurve 114 entlang der Strecke x, die orthogonal zur Mittellinie 98 des Abschnitts $90_i$ des Blutgefäßes 88 verläuft, in der Fig. 7C dargestellt.

Den Abschnitt $90_i$ des Blutgefäßes 88 beschreibt das Blutgefäß-Modell $M_B^Q$ als einen Strömungskanal 94 mit einem kreisrunden Querschnitt, der den Durchmesser D hat und durch eine Wand 95 mit der Wandstärke d begrenzt ist. Der Durchmesser D des Strömungskanals 94 sowie die Wandstärke d des Blutgefäß-Modells $MB$ werden anhand eines Kriteriums in Bezug auf den Intensitätsverlauf I(x) orthogonal zur Mittellinie 98 des Abschnitts $90_i$ des Blutgefäßes 88 in einem oder mehreren der bereitgestellten Bilder $80_1$, $80_2$, $80_3$, $80_4$, ... ermittelt.

Das Kriterium in Bezug auf den Intensitätsverlauf I(x) für das Bestimmen des Durchmessers D sowie der Wandstärke d des Blutgefäß-Modells $M_B^Q$ kann z. B. die Krümmung des Intensitätsverlaufs I(x) orthogonal zur Mittellinie 98 des Strömungskanals 94 sein, wobei eine Grenze zwischen Strömungskanal 94 und Wand 95 an den sogenannten Strömungskanal-Randpunkten 116 definiert wird, an denen die Krümmung in Form der zweiten Ableitung des Intensitätsverlaufs I(x) von der Mittellinie 98 nach außen betrachtet ein Minimum erreicht und die Intensität I(x) den Intensitätswert 115 annimmt.

Dieses Kriterium ist dadurch motiviert, dass die Erfinder Bilder eines Materials mit bekannter Wandstärke, hier eines Silikonschlauchs, der mit einem blutähnlichen Medium sowie ICG-Farbstoff gefüllt war, mit einem Operationsmikroskop 12 aufgenommen und den Intensitätsverlauf I(x) orthogonal zur Mittellinie 98 des Silikonschlauchs in den erfassten Bildern $80_1$, $80_2$, $80_3$, $80_4$, ... untersucht haben. Dies wurde für verschiedene Durchmesser des Silikonschlauchs sowie verschiedene Anordnungen dieses unter dem Operationsmikroskop 12 wiederholt. Die Erfinder haben dabei festgestellt, dass sich insbesondere die Krümmung des Intensitätsverlaufs I(x) orthogonal zur Mittellinie des Silikonschlauchs in den erfassten Bildern $80_1$, $80_2$, $80_3$, $80_4$, ... als ein Kriterium zur Bestimmung des Durchmessers D des Strömungskanals 94 eignet. Die Krümmung des Intensitätsverlaufs I(x) wird dabei in Form der zweiten Ableitung des Intensitätsverlaufs I(x) ermittelt. Die Grenze zwischen Strömungskanal 94 und Wand 95 entspricht bei diesem Vorgehen denjenigen Punkten, an denen die Krümmung des Intensitätsverlaufs I(x) von der Mittellinie 98 nach außen betrachtet ein Minimum erreicht.

Um die Grenze zwischen Strömungskanal 94 und Wand 95 zu bestimmen, ermittelt das Computerprogramm deshalb jeweils für Punkte auf der Mittellinie 98 des Abschnitts $90_i$ des Blutgefäßes 88 die Strömungskanal-Randpunkte 116 als die ersten beiden Punkte 115, an denen die Krümmung des Intensitätsverlaufs I(x) in Form der zweiten Ableitung orthogonal zur Mittellinie 98 ein Minimum aufweist. Aus den Abständen dieser beiden Strömungskanal-Randpunkte 116 wird der lokale Durchmesser $D_L$ an dem jeweiligen Punkt auf der Mittellinie 98 ermittelt. Durch Mittelung der lokalen Durchmesser $D_L$ für alle Punkte auf der Mittellinie 98 wird der Durchmesser D des Strömungskanals 94 bestimmt. Der Abstand zwischen einem Strömungskanal-Randpunkt 116 und dem Rand des segmentierten Abschnitts 90 des Blutgefäßes 88 entspricht dann der lokalen Wandstärke d_L. Diese wird ebenfalls für alle Punkte entlang der Mittellinie 98 gemittelt und daraus die Wandstärke d des Abschnitts 90 des Blutgefäßes 88 bestimmt. Durch ein Verbinden der Strömungskanal-Randpunkte 116 wird eine Segmentierung 96 des Strömungskanals 94 bestimmt.

Das Computerprogramm zur Bestimmung des Blutvolumenflusses I_{Bi} durch einen Abschnitt i eines Blutgefäßes 88 in dem Operationsbereich 36 anhand eines Fluorophors enthält zusätzlich zu dem Blutgefäß-Modell $M_B^Q$ ein Fluidströmungs-Modell $M_F^Q$, das eine lokale Strömungsgeschwindigkeit 122 an unterschiedlichen Positionen über den freien Querschnitt Q des Strömungskanals 94 in dem Blutgefäß-Modell $M_B^Q$ beschreibt.

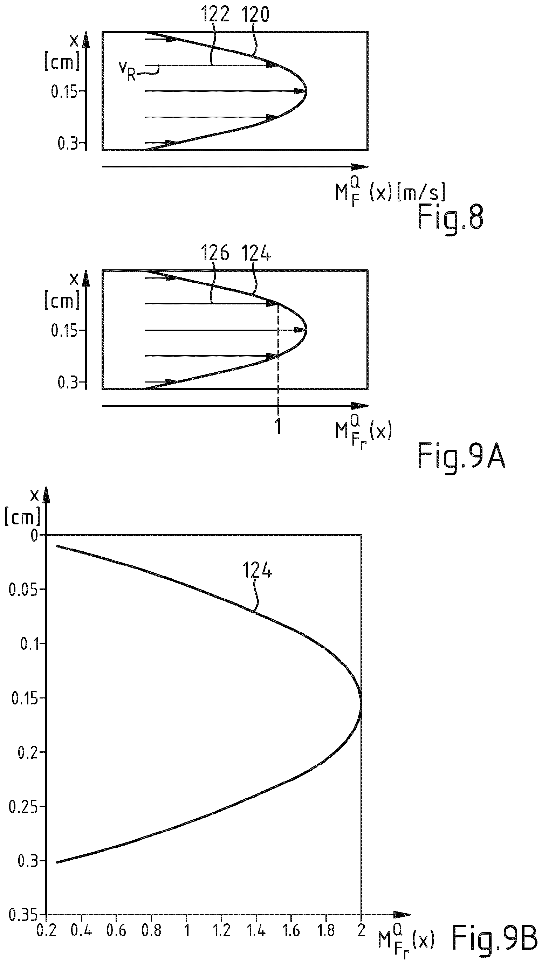

Die Fig. 8 zeigt ein Strömungsprofil 120 mit unterschiedlichen lokalen Strömungsgeschwindigkeiten 122 entlang des Durchmessers D des Querschnitts Q des Strömungskanals 94 des Blutgefäß-Modells $M_B^Q$ aus der Fig. 3. Das Fluidströmungs-Modell $M_F^Q$ des Computerprogramms beschreibt hier das Strömungsprofil 120 einer laminaren Fluidströmung durch den Strömungskanal 94 des Blutgefäß-Modells $M_B^Q$. Es ist auf einer Sehne des Querschnitts Q des Strömungskanals 94, nämlich entlang des Durchmessers D, definiert und weist die folgende Abbildungsvorschrift auf:

$$M_F^Q : [0, D] \rightarrow \mathbb{R}.$$

Das Fluidströmungs-Modell $M_F^Q$ ordnet also jeder Position entlang des Durchmessers D des Querschnitts Q des Strömungskanals 94 die lokale Strömungsgeschwindigkeit 122 an dieser Position zu.

Die Fig. 9A und die Fig. 9B zeigen jeweils ein relatives Fluidströmungs-Modell $M_{Fr}^Q$, das ein relatives Strömungsprofil 124 einer laminaren Fluidströmung durch den Strömungskanal 94 des Blutgefäß-Modells MB angibt.

Es beschreibt eine lokale relative Strömungsgeschwindigkeit 126 an unterschiedlichen Positionen über den Durchmesser D des Querschnitts Q des Strömungskanals 94 in dem Blutgefäß-Modell $M_B^Q$ in der Fig. 3 in Relation zu einer Referenz-Strömungsgeschwindigkeit v_R. Die Referenz-Strömungsgeschwindigkeit v_R wird aus den in der Fig. 8 angegebenen lokalen Strömungsgeschwindigkeiten 122 bestimmt, insbesondere durch Auswahl einer bestimmten Strömungsgeschwindigkeit in dem Strömungsprofil 124 wie in der Fig. 9A gezeigt oder durch Mittelung aller lokalen Strömungsgeschwindigkeiten 122 in dem Strömungsprofil 120 wie in der Fig. 9B zu sehen. Um ein relatives Fluidströmungs-Modell $M_{Fr}^Q$ mit relativem Strömungsprofil 124 anzugeben, werden die Werte des Strömungsprofils 120 in der Fig. 8 durch den Wert der Referenz-Strömungsgeschwindigkeit v_R dividiert. Das in der Fig. 9B gezeigte Fluidströmungs-Modell $M_F^Q$ in Form eines relativen Fluidströmungs-Modells $M_{Fr}^Q$ beschreibt einen laminaren Fluss zu einem Durchmesser D von 3 mm mit einem relativen Strömungsprofil 124 zu einer Referenz-Strömungsgeschwindigkeit v_R in Form des Mittelwerts über alle lokalen Strömungsgeschwindigkeiten 122. Das relative Strömungsprofil 124 hat hier die Form einer Parabel

$$M_F^Q(x) = a(x - b)^2 + c$$

mit

$$a = \frac{-8}{D^2}, \quad b = \frac{D}{2}, \quad c = 2.$$

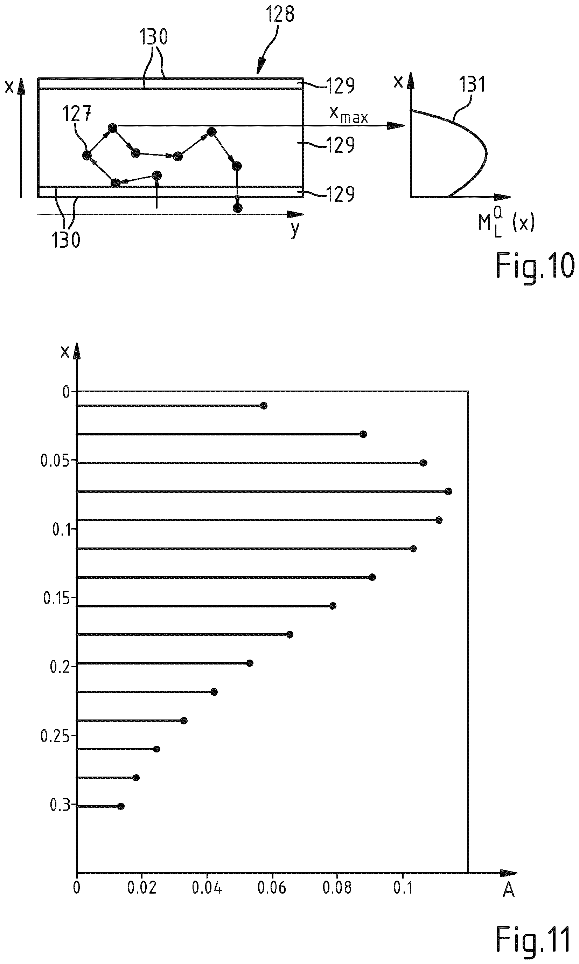

Das Computerprogramm zur Bestimmung des Blutvolumenflusses I_{Bi} durch einen Abschnitt i eines Blutgefäßes 88 in dem Operationsbereich 36 anhand eines Fluorophors enthält außerdem ein Fluoreszenzlicht-Modell $M_L^Q : Q \rightarrow \mathbb{R}$, das eine räumliche Wahrscheinlichkeitsdichte für die Intensität des remittierten Lichts an unterschiedlichen Positionen über den freien Querschnitt Q des Strömungskanals 94 in dem Blutgefäß-Modell $M_B^Q$ beschreibt, das von einem mit Fluorophor versetzten, den freien Querschnitt Q des Strömungskanals 94 in dem Blutgefäß-Modell $M_B^Q$ durchströmenden Fluid bei Bestrahlung mit Fluoreszenanregungslicht ausgesendet wird. Das Fluoreszenzlicht-Modell $M_L^Q$ wird mittels einer Monte Carlo Simulation zur Propagation von Photonen 127 in dem Blutgefäß-Modell $M_B^Q$ bestimmt, wie in der Fig. 10 gezeigt und in der oben genannten Publikation von L. Wang und S. Jacques angegeben. Das Blutgefäß-Modell $M_B^Q$ wird dabei als Schichtmodell 128 mit drei Schichten angenommen: Blutgefäßwand - Strömungskanal - Blutgefäßwand. Das Schicht-modell 128 wird mit Licht bestrahlt und der Pfad der Photonen 127 innerhalb des Schichtmodells 128 verfolgt. Die Photonen 127 werden dabei als an Streuzentren gestreute Teilchen angenommen, wobei die Streuzentren im Strömungskanal 94 und in der Wand 95 des Blutgefäß-Modells $M_B^Q$ eine jeweils charakteristische Streuzentrenverteilung aufweisen. Bei dem Auftreffen auf ein Streuzentrum werden die Photonen 127 mit einer bestimmten Wahrscheinlichkeit gestreut und mit einer anderen Wahrscheinlichkeit absorbiert. Zur Bestimmung des Fluoreszenzlicht-Modells $M_L^Q$ wird für jedes remittierte Photon 127, welches das Schichtmodell 128 durch dieselbe Schicht wieder verlässt, durch die es in das Schichtmodell 128 eingetreten ist, ermittelt, welche maximale Eindringtiefe $x_{max}$ dieses Photon 127 in dem Schichtmodell 128 erreicht hat. Die verschiedenen Eindringtiefen der Photonen 127 in das Schichtmodell 128 entsprechen dabei dem Durchmesser D des Querschnitts Q des Strömungskanals 94. Bei der Implementierung ist es von Vorteil, den Durchmesser D in n gleich große Teilstrecken zu unterteilen, z. B. für n = 100.

Der Graph 131 in Fig. 10 zeigt für verschiedene Eindringtiefen x entlang des Durchmessers D den Anteil der aus dem Schichtmodell 128 remittierten Photonen 127, deren maximale Eindringtiefe $x_{max}$ dem Wert x entspricht. Da das Fluoreszenzlicht-Modell $M_L^Q$ eine Wahrscheinlichkeitsdichte darstellt, gilt:

$$\int_Q M_L^Q(x)dx = 1.$$

Diese Wahrscheinlichkeitsdichte ist gleichzeitig ein Maß für die Intensität des remittierten Lichts an unterschiedlichen Positionen über den freien Querschnitt Q des Strömungskanals 94 in dem Blutgefäß-Modell $M_B^Q$ , da die Wahrscheinlichkeit dafür, dass ein Photon 127 eine bestimmte Eindringtiefe erreicht, auch ein Maß für die Intensität des aus dieser Tiefe remittierten Lichts ist.

Die Fig. 11 zeigt ein anhand einer oben beschriebenen Monte Carlo Simulation ermitteltes Fluoreszenzlicht-Modell $M_L^Q$ . Auf der vertikalen Achse ist die Eindringtiefe x entlang des Durchmessers D des Strömungskanals abgetragen.

Auf der horizontalen Achse ist der Anteil A der in der Fig. 10 gezeigten Photonen 127 abgetragen, die während der Simulation maximal die Eindringtiefe x erreicht haben und durch dieselbe Wand 95 des Blutgefäß-Modells $M_B^Q$ wieder ausgetreten sind, durch die sie in das Modell eingetreten sind.

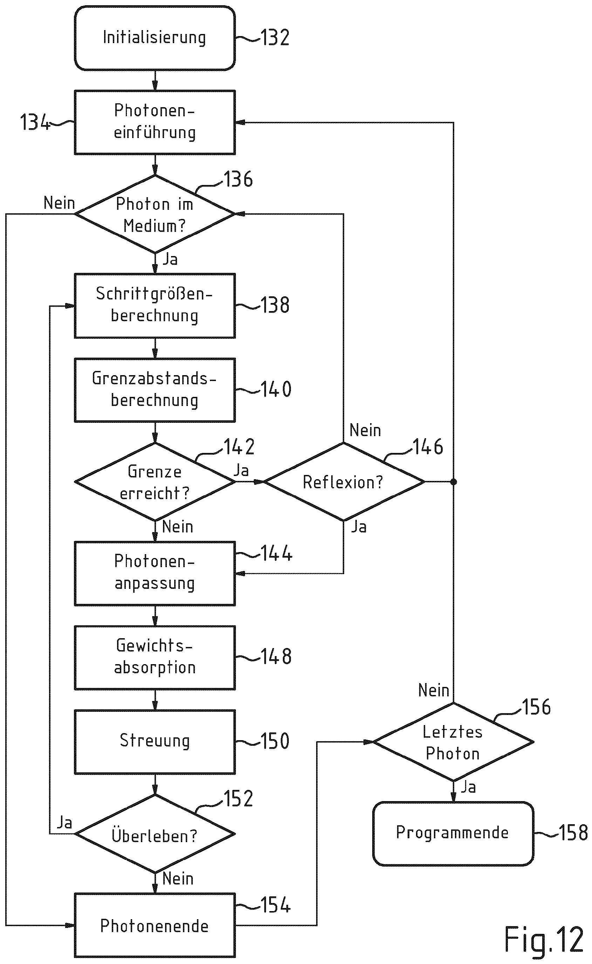

Die Fig. 12 zeigt ein Ablaufdiagramm für die Simulation der Photonen-Bewegung in dem Schichtmodell 128 wie in der oben genannten Publikation von Wang und Jacques beschrieben. In einem Schritt der Initialisierung 132 wird das Programm initialisiert und die Parameter geladen. Danach wird in einem Schritt der Photoneneinführung 134 die Simulation eines Photons 127 mit einem festgelegten Ausgangsgewicht gestartet. In einer Abfrage 136 wird überprüft, ob das Photon 127 in das Schichtmodell 128 eingetreten ist oder ob es durch spekulare Reflexion schon vor dem Eintritt in das Schichtmodell 128 reflektiert wurde. Ist das Photon 127 in das Schichtmodell 128 eingetreten und befindet sich in einer Schicht 129, wird in einem Schritt der Schrittgrößenberechnung 138 eine Schrittgröße des Photons in Abhängigkeit von den Eigenschaften des Mediums in dieser Schicht 129 anhand einer Wahrscheinlichkeitsverteilung über die freie Weglänge des Photons 127 bestimmt. In einem Schritt der Grenzabstandsbestimmung 140 wird der Abstand zur Schichtgrenze 130 der nächsten Schicht 129 in der Bewegungsrichtung des Photons 127 ermittelt. In einer folgenden Abfrage 142 wird überprüft, ob das Photon 127 in dem nächsten Schritt mit der ermittelten Schrittgröße in seiner Bewegungsrichtung eine Schichtgrenze 130 der Schicht 129 erreichen oder überschreiten würde. Ist dies nicht der Fall, so wird der Ort des Photons 127 in einem Schritt der Photonen-Anpassung 144 anhand seiner Schrittgröße angepasst. Erreicht oder überschreitet das Photon 127 aber eine Schichtgrenze 130, so wird in einer Abfrage 146 überprüft, ob das Photon 127 an dieser Schichtgrenze 130 reflektiert oder in die nächste Schicht 129 übertragen wird. Wird das Photon 127 in die nächste Schicht 129 übertragen, so werden Parameter des Photons 127 wie z. B. die Schrittgröße an die nächste Schicht 129 angepasst und die oben beschriebenen Schritte ab der Abfrage 136, ob sich das Photon 127 im Medium befindet, wiederholt. Wird das Photon 127 an der Schichtgrenze 130 der Schicht 129 reflektiert, so wird anhand der Parameter des Photons 127 der Ort und die Richtung des Photons in dem Schritt der Photonen-Anpassung 144 angepasst. In einem Schritt der Gewichtsabsorption 148 wird das Gewicht des Photons 127 aufgrund von Absorption durch den Interaktionsort verringert. Dabei wird ein Teil des aktuellen Gewichts des Photons 127 an dem lokalen Ort des Schichtmodells 128 deponiert und das Gewicht des Photons 127 angepasst. Nach der Bewegung des Photons 127 und der Verringerung des Gewichts wird das Photon 127 in einem Schritt der Streuungsberechnung 150 anhand der Eigenschaften des Mediums und verschiedener statistisch ermittelter Winkel gestreut und seine Parameter angepasst. Um Photonen 127 mit sehr geringem Gewicht, deren weitere Bewegung nur noch sehr geringe Auswirkungen auf das Modell haben, zu terminieren, wird in einer Abfrage 152 anhand von Zufallswertes überprüft, ob das Photon 127 noch weiter in der Simulation überlebt oder ob es in einem Schritt Photonenende 154 terminiert werden soll. Handelt es sich um das letzte Photon 156 in der Simulation, so wird in einem letzten Schritt des Programmendes 158 das Programm beendet. Ansonsten wird in dem Schritt der Photoneneinführung 134 das nächste Photon 127 in das Schichtmodell 128 eingeführt.

Zur Ermittlung des Fluoreszenzlicht-Modells $M_L^Q$ anhand dieser Simulation haben die Erfinder die Bewegung von 1.000.000 Photonen 127 ermittelt und dabei die folgenden Parameter für ein Schichtmodell 128 mit drei Schichten 129 verwendet:

|  | Blutgefäßwand | Strömungskanal |
|---|---|---|
| Absorptionskoeffizient $\mu_a$ | 2.25 cm$^{-1}$ | 7.38 cm$^{-1}$ |
| Streukoeffizient $\mu_s$ | 200 cm$^{-1}$ | 713 cm$^{-1}$ |
| Brechungsindex n | 1.44 | 1.38 |
| Anisotropie g | 0.99 | 0.99 |

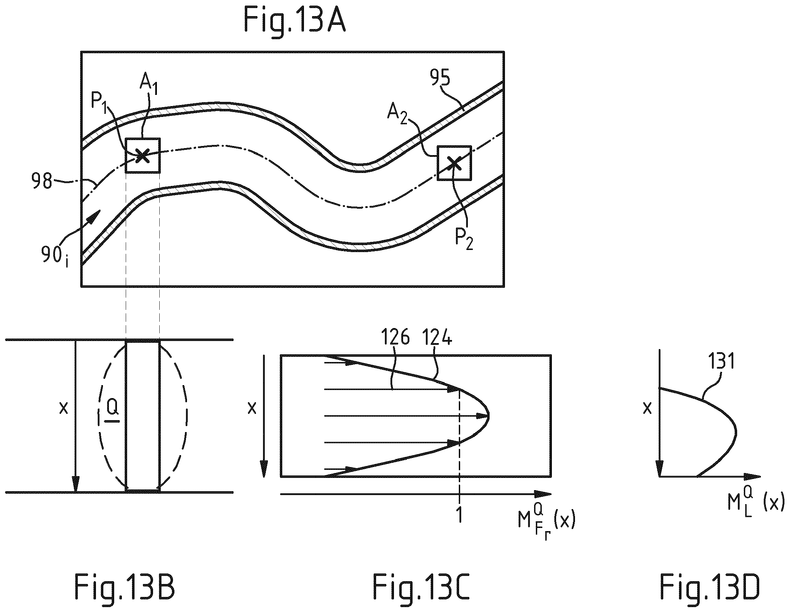

Die Fig. 13A zeigt einen horizontalen Querschnitt eines Abschnitts $90_i$ eines Blutgefäßes 88 mit einem Anfangspunkt $P_1$ und einem Endpunkt $P_2$ auf der Mittellinie 98 des Abschnitts $90_i$ des Blutgefäßes 88 sowie einen ersten Ausschnitt $A_1$, der den Anfangspunkt $P_1$ enthält, und einen zweiten Ausschnitt $A_2$, der den Endpunkt $P_2$ enthält.

Die Fig. 13B zeigt zu dem ersten Ausschnitt $A_1$ in der Fig. 13A einen vertikalen Querschnitt des Abschnitts $90_i$ des Blutgefäßes 88 entlang der Eindringtiefe $x \in D$. Zu jeder Eindringtiefe x ist die zugehörige lokale relative Strömungsgeschwindigkeit 126 durch das relative Fluidströmungs-Modell $M_{Fr}^Q$ in der Fig. 13C sowie der Anteil an Photonen 127, die aus der jeweiligen Eindringtiefe x remittiert werden, durch das Fluoreszenzlicht-Modell $M_L^Q$ in der Fig. 13D angegeben. Das relative Fluidströmungs-Modell $M_{Fr}^Q$ ist dabei in Relation zur mittleren Strömungsgeschwindigkeit

$$v_R = v_{Mittel} = \frac{\int_Q M_F^Q(x)dx}{\int_Q dx}$$

des Fluidströmungs-Modells $M_F^Q$ angegeben. Die korrigierte Strömungsgeschwindigkeit $v_{korrigiert}$ in Form der zu erwartenden mittleren Strömungsgeschwindigkeit $\overline{v_{Mittel}}$ in dem Abschnitt des Blutgefäßes lässt sich aus der in dem Abschnitt des Blutgefäßes beobachteten Strömungsgeschwindigkeit $v_{beobachtet}$ anhand des relativen Fluidströmungs-Modells $M_{Fr}^Q$ sowie des Fluoreszenzlicht-Modells $M_L^Q$ wie folgt bestimmen:

$$v_{korrigiert} := \overline{v_{Mittel}} = v_{beobachtet} \cdot k_{Mittel}$$

mit dem Korrekturfaktor

$$k_{Mittel} := \frac{1}{\int_Q M_{Fr}^Q(x) \ M_L^Q(x) dx}.$$

Für das in der Fig. 9B gezeigte relative Fluidströmungs-Modell $M_{Fr}^Q$ zu der Referenz-Strömungsgeschwindigkeit $v_{Mittel}$ und das in dem Graph 131 der Fig. 10 gezeigte Fluoreszenzlicht-Modell $M_L^Q$ ergibt sich so für einen Durchmesser D von 3 mm z. B. der Korrekturfaktor $k_{Mittel}$ = 0.68.

Um Rechenzeit zu sparen, wird der Korrekturfaktor $k\_v_R$ für verschiedene Modellparameter, z. B. für verschiedene Durchmesser D des Strömungskanals 94, vorab berechnet und in einem Look-up-Table (LUT) abgespeichert.

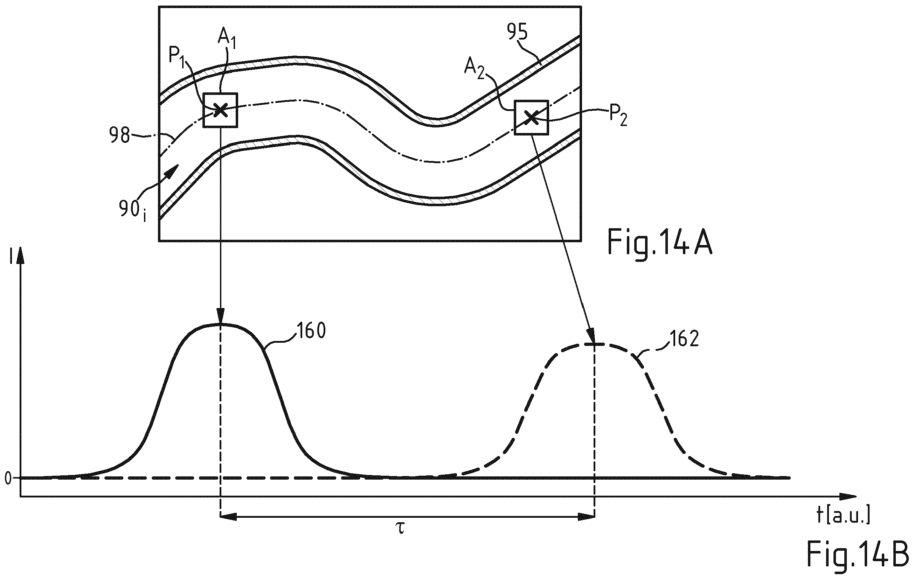

Die Fig. 14A zeigt einen horizontalen Querschnitt eines Abschnitts $90_i$ eines Blutgefäßes 88.

Die Fig. 14B zeigt eine Berechnung der in dem Abschnitt des Blutgefäßes beobachteten Geschwindigkeit $v_{beobachtet}$ in dem Abschnitt $90_i$ des Blutgefäßes 88. Dazu wird eine Zeitentwicklung 160 der Intensität I in dem ersten Ausschnitt $A_1$ und eine Zeitentwicklung 162 der Intensität in dem zweiten Ausschnitt $A_2$ über die Mehrzahl der bereitgestellten Bilder $80_1$, $80_2$, $80_3$, $80_4$, ... betrachtet. Dafür werden die Intensitätswerte I(t) über die Pixel in dem jeweiligen Ausschnitt $A_1$, $A_2$ gemittelt. An die diskreten Intensitätswerte I(t) wird eine kontinuierliche Funktion 106 in Form einer Gammafunktion gefittet, um Zwischenwerte zu erhalten und auch bei niedriger Bildrate eine möglichst genaue in dem Abschnitt des Blutgefäßes beobachtete Geschwindigkeit $v_{beobachtet}$ ermitteln zu können.

Der vorliegend durch Kreuzkorrelation bestimmte zeitliche Versatz der beiden Kurven entspricht dann der Transitzeit $\tau$. Auch die Blutflussrichtung lässt sich aus diesem Versatz ableiten. Zu bemerken ist, dass der zeitliche Versatz der beiden Kurven grundsätzlich anstatt mittels Kreuzkorrelation auch durch eine Mittelung von zeitlichen Verschiebungen von distinkten Merkmalen der beiden Kurven bestimmt werden kann.

Aus der Transitzeit $\tau$, der Länge L und dem Durchmesser D des Abschnitts $90_i$ des Blutgefäßes 88 sowie dem Korrekturfaktor $k\_v_R$ lässt sich dann der Blutvolumenfluss $I_{Bi}$ berechnen als

$$I_{Bi} = \dot{V} \doteq \left(\frac{D}{2}\right)^2 \pi \frac{L\,k}{\tau} = \left(\frac{D}{2}\right)^2 \pi \, v_{beobachtet} \, k\_v_R.$$

Zu dem Blutvolumenfluss $I_{Bi}$ wird ein Vertrauensintervall in Abhängigkeit von der Länge L, dem Durchmesser D, dem Korrekturfaktor k, der Transitzeit $\tau$ und der Form der Mittellinie 98 angegeben, wobei hierfür auch der Winkel der Teilabschnitte der Mittelline zu Gitterlinien eines Gitters des Bildsensors der Bilderfassungseinrichtung berücksichtigt wird.

Die Unsicherheit wird dabei nach DIN 1319 als die Fortpflanzung von nichtkorrelierten Eingangs-Unsicherheiten ohne Annahme einer Normalverteilung berechnet.

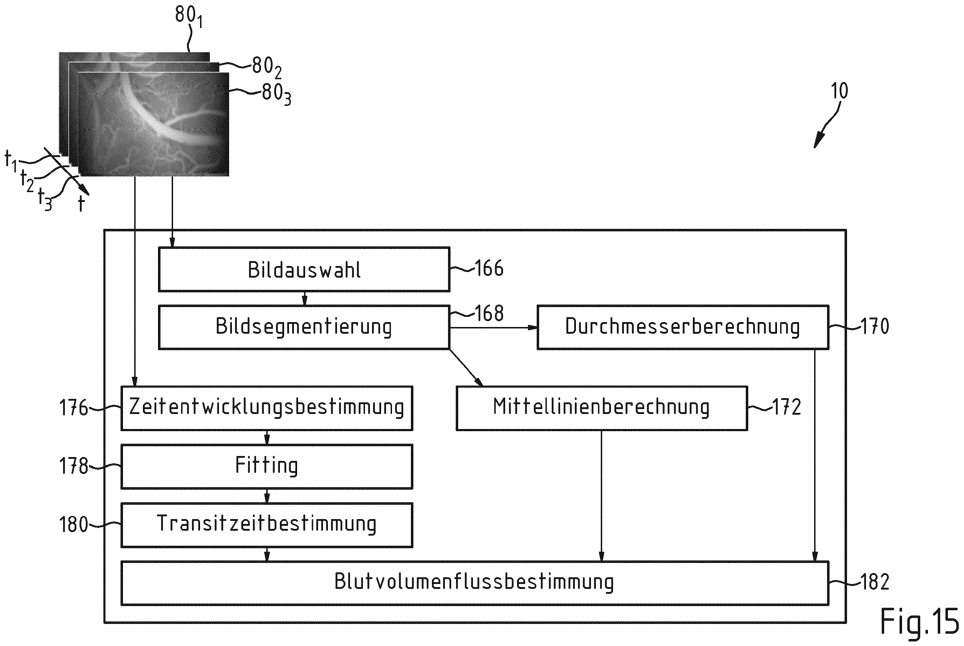

Die Fig. 15 zeigt ein Ablaufdiagramm zu einer Ausführungsform des Verfahrens 10 zur Bestimmung des Blutvolumenflusses $I_{Bi}$ in einem Abschnitt $90_i$ eines Blutgefäßes 88 in einem Operationsbereich 36 eines Patienten anhand einer Mehrzahl bereitgestellter Bilder $80_1$, $80_2$, $80_3$, $80_4$, .... In einem Schritt der Bildauswahl 166 wird ein Bild aus den bereitgestellten Bildern $80_1$, $80_2$, $80_3$, $80_4$, ... ausgewählt. Das ausgewählte Bild wird in einem Schritt der Bildsegmentierung 168 segmentiert. Aus der Segmentierung 96 wird ein Durchmesser D in einem Schritt der Durchmesserberechnung 170 bestimmt. Außerdem wird eine Mittelinie 98 des Abschnitts $90_i$ des Blutgefäßes 88 in einem Schritt der Mittellinienberechnung 172 sowie deren Länge L ermittelt.

Die Zeitentwicklung 160, 162 der Intensität in dem ersten und zweiten Abschnitt wird in einem Schritt der Zeitentwicklungsbestimmung 176 bestimmt. In einem Schritt des Fittings 178 wird eine kontinuierliche Funktion 106 an die Zeitentwicklungen 160, 162 angepasst. Aus dem Versatz zwischen den an die Zeitentwicklungen 160, 162 angepassten kontinuierlichen Funktionen 106 wird die Transitzeit $\tau$ in einem Schritt der Transitzeitbestimmung 180 berechnet. Aus den ermittelten Daten wird schließlich in einem Schritt der Blutvolumenflussbestimmung 182 der Blutvolumenfluss $I_{Bi}$ ermittelt.

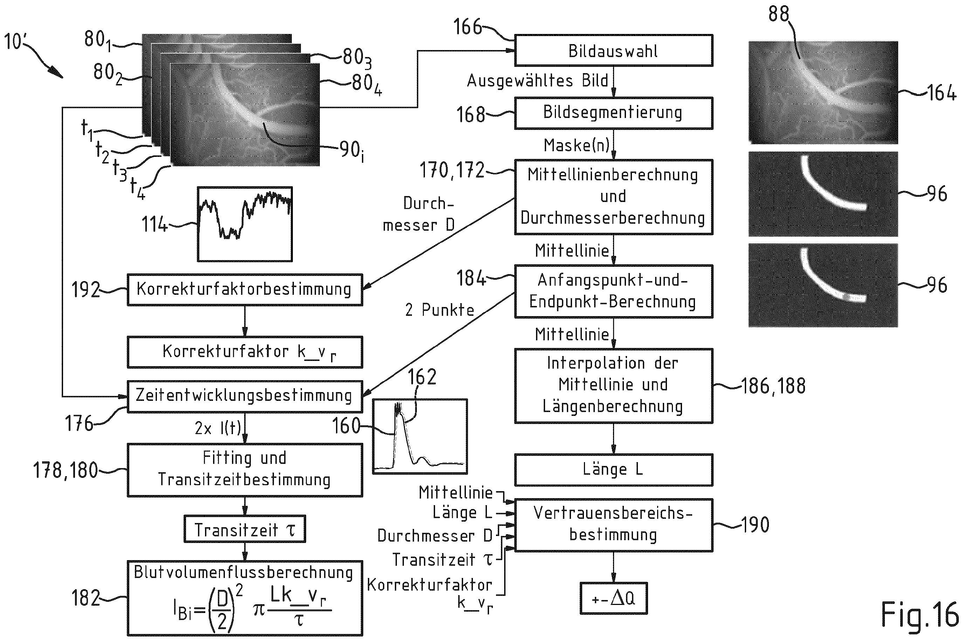

Die Fig. 16 zeigt ein Ablaufdiagramm zu einer weiteren Ausführungsform des Verfahrens 10' zur Bestimmung des Blutvolumenflusses $I_{Bi}$ in einem Abschnitt $90_i$ eines Blutgefäßes 88 in einem Operationsbereich 36. Dabei wird mit einer Bilderfassungseinrichtung 64 ein Video des Operationsbereichs 36 unter Beleuchtungslicht 46 aufgenommen, sodass der Fluoreszenzfarbstoff sichtbar ist. Das Video besteht aus einer Mehrzahl von Bildern $80_1$, $80_2$, $80_3$, $80_4$, ..., die auf Fluoreszenzlicht in Form von Beleuchtungslicht 46 mit innerhalb eines Fluoreszenzspektrums des Fluorophors liegenden Wellenlängen basieren und die den Abschnitt $90_i$ des Blutgefäßes 88 zu unterschiedlichen Aufnahmezeitpunkten zeigen. Aus der Mehrzahl der Bilder $80_1$, $80_2$, $80_3$, $80_4$, ... wird in einem Schritt der Bildauswahl 166 ein ausgewähltes Bild 164 bestimmt, welches die maximale Anzahl an farbgesättigten Pixeln aller bereitgestellten Bilder $80_1$, $80_2$, $80_3$, $80_4$, ... aufweist. Anhand eines Bildsegmentierungsverfahrens wird in einem Schritt der Bildsegmentierung 168 der Abschnitt $90_i$ des Blutgefäßes 88 in dem ausgewählten Bild 164 ermittelt. Aus der Segmentierung 96 wird in einer Mittellinienberechnung 172 eine Mittellinie 98 des Abschnitts $90_i$ des Blutgefäßes 88 berechnet. Anhand der Segmentierung 96 des Abschnitts $90_i$ des Blutgefäßes 88 und der Mittellinie 98 wird in einer Durchmesserberechnung 170 der Durchmesser D berechnet. In einer Anfangspunkt-und-Endpunkt-Berechnung 184 werden ein Anfangspunkt $P_1$ und ein Endpunkt $P_2$ auf der Mittellinie ermittelt. In einem Schritt der Interpolation 186 wird die Mittellinie 98, z. B. abschnittsweise mit einem Bezier Spline, interpoliert und anhand der interpolierten Mittelinie 98 in einer Längenberechnung 188 die Länge L des Abschnitts $90_i$ des Blutgefäßes 88 ermittelt. Anhand der Mittellinie 98, der Kurve 114 des Intensitätsverlaufs I(x) orthogonal zur Mittellinie 98 und der Segmentierung 96 des Abschnitts $90_i$ des Blutgefäßes 88 wird in einer Durchmesserberechnung 170 ein Durchmesser D des Abschnitts $90_i$ des Blutgefä-βes 88 ermittelt. Zu dem Durchmesser D wird dann anhand eines Look-up-Tables ein Korrekturfaktor $k\_v_r$ in einer Korrekturfaktorbestimmung 192 ermittelt. Außerdem wird eine charakteristische Transitzeit $\tau$ als Zeitintervall zu einer Ausbreitung des Fluorophors durch den Abschnitt $90_i$ des Blutgefäßes 88 bestimmt. Dazu wird in einer Zeitentwicklungsbestimmung 176 an zwei unterschiedlichen Ausschnitten $A_1$, $A_2$, wobei $A_1$ den Anfangspunkt $P_1$ und $A_2$ den Endpunkt $P_2$ enthält, eine Zeitentwicklung 160, 162 der Bildhelligkeit ermittelt. Dazu wird ein Mittelwert der Intensitäten in dem jeweiligen Ausschnitt $A_1$, $A_2$ über die Zeit betrachtet. An die sich ergebenden Messwerte der Zeitentwicklung 160, 162 wird jeweils eine kontinuierliche Funktion 106, z. B. eine Gamma-Funktion, in einem Schritt des Fittings 178 gefittet. Aus dem zeitlichen Versatz der sich ergebenden Kurven wird die Transitzeit $\tau$ in einer Transitzeitbestimmung 180 berechnet. Der Blutvolumenfluss $I_{Bi}$ ergibt sich schließlich aus der Länge L und dem Durchmesser D des Abschnitts $90_i$ des Blutgefäßes 88 sowie der Transitzeit $\tau$ und dem Korrekturfaktor $k\_v_r$ in einer Blutvolumenflussbestimmung 182. Zusätzlich wird ein Vertrauensbereich in einer Vertrauensbereichsbestimmung 190 anhand der berechneten Parameter ermittelt.

Zusammenfassend ist insbesondere Folgendes zu bemerken: Die Erfindung betrifft ein computerimplementiertes Verfahren 10 zur Bestimmung des Blutvolumenflusses $I_{Bi}$ durch einen Abschnitt $90_i$, i = 1, 2, 3, ... eines Blutgefäßes 88 in einem Operationsbereich 36 anhand eines Fluorophors, bei dem eine Mehrzahl von Bildern $80_1$, $80_2$, $80_3$, $80_4$, ..., die auf Fluoreszenzlicht in Form von Licht mit innerhalb eines Fluoreszenzspektrums des Fluorophors liegenden Wellenlängen basieren und die den Abschnitt $90_i$ des Blutgefäßes 88 zu unterschiedlichen Aufnahmezeitpunkten $t_1$, $t_2$, $t_3$, $t_4$, ... zeigen, bereitgestellt wird, bei dem durch Verarbeiten der bereitgestellten Bilder $80_1$, $80_2$, $80_3$, $80_4$, ... ein Durchmesser D und eine Länge L des Abschnitts $90_i$ des Blutgefäßes 88 sowie ein Zeitintervall zu einer Ausbreitung des Fluorophors durch den Abschnitt $90_i$ des Blutgefäßes 88 ermittelt wird, das eine charakteristische Transitzeit $\tau$ für das Fluorophor in dem Abschnitt $90_i$ des Blutgefäßes 88 beschreibt, wobei ein Blutgefäß-Modell $M_B^Q$ für den Abschnitt $90_i$ des Blutgefäßes 88 verarbeitet wird, welches den Abschnitt $90_i$ des Blutgefäßes 88 als einen Strömungskanal 94 mit einer Länge L, mit einer Wand 95 mit einer Wandstärke d und mit einem freien Querschnitt Q beschreibt, wobei wenigstens eines der bereitgestellten Bilder $80_1$, $80_2$, $80_3$, $80_4$, ... verarbeitet wird, welches ein Fluidströmungs-Modell $M_F^Q$ zu dem Blutgefäß-Modell $M_B^Q$ verarbeitet, das eine lokale Strömungsgeschwindigkeit 122 an unterschiedlichen Positionen über den freien Querschnitt Q des Strömungskanals 94 in dem Blutgefäß-Modell $M_B^Q$ beschreibt, welches ein Fluoreszenzlicht-Modell $M_L^Q$ verarbeitet, das eine räumliche Wahrscheinlichkeitsdichte für die Intensität des remittierten Lichts an unterschiedlichen Positionen über den freien Querschnitt Q des Strömungskanals 94 in dem Blutgefäß-Modell $M_B^Q$ beschreibt, das von einem mit Fluorophor versetzten, den freien Querschnitt Q des Strömungskanals 94 in dem Blutgefäß-Modell $M_B^Q$ durchströmenden Fluid bei Bestrahlung mit Fluoreszenzanregungslicht ausgesendet wird, und bei welchem der Blutvolumenfluss $I_{Bi}$ als ein durch den Strömungskanal 94 in dem Blutgefäß-Modell $M_B^Q$ geführter Fluidstrom bestimmt wird, der anhand des Fluidströmungs-Modells $M_F^Q$ und des Fluoreszenzlicht-Modells $M_L^Q$ aus der Länge L und dem Durchmesser D des Abschnitts $90_i$ des Blutgefäßes 88 sowie der charakteristischen Transitzeit $\tau$ für das Fluorophor in dem Abschnitt $90_i$ des Blutgefäßes 88 berechnet wird.

**Bezugszeichenliste**

| | |
|---|---|
| 10, 10' | Verfahren |
| 12 | Operationsmikroskop |
| 14 | System zur Bestimmung des Blutvolumenflusses |
| 20 | Mikroskop-Hauptobjektiv |
| 22 | Mikroskop-Grundkörper |
| 24 | Vergrößerungssystem |
| 26 | linker Beobachtungsstrahlengang |
| 28 | rechter Beobachtungsstrahlengang |
| 30 | Binokulartubus |
| 32 | Okularlinse |
| 34 | Tubuslinse |
| 36 | Operationsbereich |
| 37 | Gehirn |
| 38 | linkes Beobachterauge |
| 40 | rechtes Beobachterauge |
| 42 | Beleuchtungseinrichtung |
| 44 | Beleuchtungsstrahlengang |
| 46 | Beleuchtungslicht |
| 48 | Xenon-Lichtquelle |
| 50 | Linse |
| 52 | Lichtleiter |
| 54 | Beleuchtungsobjektiv |
| 56 | Beleuchtungsfilter |
| 59 | Pfeil |
| 60 | Beobachtungsfilter für den linken Beobachtungsstrahlengang |
| 62 | Beobachtungsfilter für den rechten Beobachtungsstrahlengang |
| 64 | Bilderfassungseinrichtung |
| 66 | Auskopplungs-Strahlteiler |
| 68 | optische Achse |
| 70 | Bildsensor |
| 71 | Bereich |
| 72 | Rechnereinheit |
| 74 | Eingabeeinheit |
| 76 | Programmspeicher |
| 78 | Bildschirm |
| $80_1$ | Bild 1 |
| $80_2$ | Bild 2 |
| $80_3$ | Bild 3 |
| $80_4$ | Bild 4 |
| 82 | Display |
| 84 | Linse |
| 86 | Strahlteiler |
| 88 | Blutgefäß |
| 89 | Verzweigung |
| 90, $90_i$, i = 1, 2, 3, ... | Abschnitt |
| 91 | Zylinderachse |
| 92 | Pfeil |
| 93 | Pfeil |
| 94 | Strömungskanal |
| 95 | Wand |
| 96 | Segmentierung |
| $97_i$ | Bildabschnitt |
| 98 | Mittellinie |
| 99 | Mittellinienabschnitt |

| 100 | nachverarbeitete Mittellinie |
|---|---|
| 102 | diskretisierte Mittellinie |
| 104 | Polygonzug |
| 106 | kontinuierliche Funktionen |
| 108 | Pixelnachbarschaft |
| 110 | L-förmige Verbindungsstruktur |
| 112 | diagonale Verbindungsstruktur |
| 114 | Kurve |
| 115 | Intensitätswert |
| 116 | Strömungskanal-Randpunkt |
| 120 | Strömungsprofil |
| 122 | lokale Strömungsgeschwindigkeit |
| 124 | relatives Strömungsprofil |
| 126 | lokale relative Strömungsgeschwindigkeit |
| 127 | Photon |
| 128 | Schichtmodell |
| 129 | Schicht |
| 130 | Schichtgrenze |
| 131 | Graph |
| 132 | Initialisierung |
| 134 | Photoneneinführung |
| 136 | Abfrage |
| 138 | Schrittgrößenberechnung |
| 140 | Grenzabstandsbestimmung |
| 142 | Abfrage |
| 144 | Photonen-Anpassung |
| 146 | Abfrage |
| 148 | Gewichtsabsorption |
| 150 | Streuungsberechnung |
| 152 | Abfrage |
| 154 | Photonenende |
| 158 | Programmende |
| 160 | Zeitentwicklung der Intensität I(t) in dem ersten Ausschnitt |
| 162 | Zeitentwicklung der Intensität I(t) in dem zweiten Ausschnitt |
| 164 | ausgewähltes Bild |
| 166 | Bildauswahl |
| 168 | Bildsegmentierung |
| 170 | Durchmesserberechnung |
| 172 | Mittellinienberechnung |
| 176 | Zeitentwicklungsbestimmung |
| 178 | Fitting |
| 180 | Transitzeitbestimmung |
| 182 | Blutvolumenflussbestimmung |
| 184 | Anfangspunkt-und-Endpunkt-Berechnung |
| 186 | Interpolation |
| 188 | Längenberechnung |
| 190 | Vertrauensbereichsbestimmung |
| 192 | Korrekturfaktorbestimmung |

| $I_B$ | Blutvolumenfluss durch Blutgefäß |
|---|---|
| $I_{Bi}$ | Blutvolumenfluss durch i-ten Abschnitt eines Blutgefäßes |
| ICG | Indocyaningrün |
| $MB$ | Blutgefäß-Modell |
| $M_F^Q$ | Fluidströmungs-Modell |

$M_{Fr}^{Q}$ relatives Fluidströmungs-Modell

$M_{L}^{Q}$ Fluoreszenzlicht-Modell

$t_1, t_2, t_3, t_4$ Aufnahmezeiten

L Länge des Abschnitts des Blutgefäßes

Q Querschnitt des Strömungskanals

a Zylinderachse

D Durchmesser

$D_L$ lokaler Durchmesser

G Gesamtdurchmesser

$G_L$ lokaler Gesamtdurchmesser

d Wandstärke

$d_L$ lokale Wandstärke

$P_1$ Anfangspunkt

$P_2$ Endpunkt

$v_R$ Referenz-Strömungsgeschwindigkeit

$A_1$ erster Ausschnitt

$A_2$ zweiter Ausschnitt

$\tau$ Transitzeit

$k\_v_R$ Korrekturfaktor zur Referenz-Strömungsgeschwindigkeit $v_R$

$v_{Modell}$ in dem Blutgefäß-Modell beobachtete Strömungsgeschwindigkeit

$v_{beobachtet}$ in dem Abschnitt des Blutgefäßes beobachtete Strömungsgeschwindigkeit

**Patentansprüche**

1. Computerimplementiertes Verfahren (10) zur Bestimmung des Blutvolumenflusses ($I_{Bl}$) durch einen Abschnitt ($90_1$, $90_2$, $90_3$, ...) eines Blutgefä-βes (88) in einem Operationsbereich (36) anhand eines Fluorophors,

bei dem eine Mehrzahl von Bildern ($80_1$, $80_2$, $80_3$, $80_4$,...), die auf Fluoreszenzlicht in Form von Licht mit innerhalb eines Fluoreszenzspektrums des Fluorophors liegenden Wellenlängen basieren und die den Abschnitt ($90_1$, $90_2$, $90_3$, ...) des Blutgefäßes (88) zu unterschiedlichen Aufnahmezeiten ($t_1$, $t_2$, $t_3$, $t_4$, ...) zeigen, bereitgestellt wird,
bei dem durch Verarbeiten der bereitgestellten Bilder ($80_1$, $80_2$, $80_3$, $80_4$,...) ein Durchmesser (D) und eine Länge (L) des Abschnitts (90) des Blutgefäßes (88) sowie ein Zeitintervall zu einer Ausbreitung des Fluorophors durch den Abschnitt ($90_1$, $90_2$, 90s, ...) des Blutgefäßes (88) ermittelt wird, das eine charakteristische Transitzeit ($\tau$) für das Fluorophor in dem Abschnitt ($90_1$, $90_2$, 90s, ...) des Blutgefäßes (88) beschreibt,

bei dem ein Blutgefäß-Modell ( $M_{B}^{Q}$ ), welches den Abschnitt ($90_1$, $90_2$, $90_3$, ...) des Blutgefäßes (88) als einen Strömungskanal (94) mit einer Länge (L), mit einer Wand (95) mit einer Wandstärke (d) und mit einem freien Querschnitt Q beschreibt, an wenigstens eines der bereitgestellten Bilder ($80_1$, $80_2$, $80_3$, $80_4$,...) mittels Bild-verarbeitung angepasst wird;

bei dem ein Fluidströmungs-Modell $M_{F}^{Q}$ zu dem angepassten Blutgefäß-Modell ( $M_{B}^{Q}$ ) bereitgestellt wird, das eine lokale Strömungsgeschwindigkeit (122) an unterschiedlichen Positionen über den freien Querschnitt Q des Strömungskanals (94) in dem angepassten Blutgefäß-Modell ( $M_{B}^{Q}$ ) beschreibt;
**dadurch gekennzeichnet, dass**

ein Fluoreszenzlicht-Modell $M_{L}^{Q}$ bereitgestellt wird, das eine räumliche Wahrscheinlichkeitsdichte für die Intensität des remittierten Lichts an unterschiedlichen Positionen über den freien Querschnitt Q des Strömungskanals (94) in dem angepassten Blutgefäß-Modell ( $M_{B}^{Q}$ ) beschreibt, das von einem mit Fluorophor versetzten, den freien Querschnitt Q des Strömungskanals (94) in dem angepassten Blutgefäß-Modell ( $M_B^Q$ ) durchströmenden Fluid bei Bestrahlung mit Fluoreszenzanregungslicht ausgesendet wird; und

der Blutvolumenfluss ($I_{BI}$) als ein durch den Strömungskanal (94) in dem angepassten Blutgefäß-Modell ( $M_B^Q$ ) geführter Fluidstrom bestimmt wird, der anhand des bereitgestellten Fluidströmungs-Modells $M_F^Q$ und des bereitgestellten Fluoreszenzlicht-Modells Mf aus der Länge (L) und dem Durchmesser (D) des Abschnitts ($90_1$, $90_2$, $90_3$, ...) des Blutgefäßes (88) sowie der charakteristischen Transitzeit ($\tau$) für das Fluorophor in dem Abschnitt ($90_1$, $90_2$, $90_3$, ...) des Blutgefäßes (88) berechnet wird.

2. Computerimplementiertes Verfahren nach Anspruch 1, **dadurch gekennzeichnet, dass** die Mehrzahl von Bildern (801, 802, 803, 804, ...) mit einer Bilderfassungseinrichtung (64) erfasst werden, wobei während der Bestimmung des Blutvolumenflusses ($I_{BI}$) Parameter der Bilderfassungseinrichtung (64), des Blutgefäß-Modells ( $M_B^Q$ ), des Fluidströmungs-Modells $M_F^Q$ und des Fluoreszenzlicht-Modells $M_L^Q$ nicht verändert werden.

3. Computerimplementiertes Verfahren nach Anspruch 1 oder 2, **dadurch gekennzeichnet, dass** das Blutgefäß-Modell ( $M_B^Q$ ) ein Hohlzylinder mit der Länge (L), dem Durchmesser (D) und der Wandstärke (d) ist und/oder

   dass das Fluidströmungs-Modell $M_F^Q$ eine laminare Fluidströmung durch den Strömungskanal (94) des Blutgefäß-Modells ( $M_B^Q$ ) beschreibt.

4. Computerimplementiertes Verfahren nach einem der Ansprüche 1 bis 3, **dadurch gekennzeichnet, dass** das Fluoreszenzlicht-Modell M2 auf einer Simulation einer Bestrahlung des Blutgefäß-Modells ( $M_B^Q$ ) mit Fluoreszenzanregungslicht beruht, bei der Photonen (127) als an Streuzentren gestreute Teilchen angenommen werden, wobei die Streuzentren im Strömungskanal (94) und in der Wand (95) des Blutgefäß-Modells ( $M_B^Q$ ) eine jeweils charakteristische Streuzentrenverteilung aufweisen.

5. Computerimplementiertes Verfahren nach Anspruch 4, **dadurch gekennzeichnet, dass** das Urbild des Fluoreszenzlicht-Modells $M_L^Q$ einer Sehne des freien Querschnitts Q des Blutgefäß-Modells ( $M_B^Q$ ) entspricht, welche die Eindringtiefe der Photonen (127) in das Blutgefäß-Modell ( $M_B^Q$ ) bei Bestrahlung mit Fluoreszenzanregungslicht darstellt und dass das Fluoreszenzlicht-Modell $M_L^Q$ die Eindringtiefe x auf den Anteil der aus dem Blutgefäß-Modell ( $M_B^Q$ ) remittierten Photonen (127) abbildet, deren maximale Eindringtiefe in dem Blutgefäß-Modell ( $M_B^Q$ ) während der Simulation dem Wert x entspricht.

6. Computerimplementiertes Verfahren nach einem der Ansprüche 1 bis 5, **dadurch gekennzeichnet, dass** das Fluidströmungs-Modell $M_F^Q$ ein relatives Fluidströmungs-Modell $M_{Fr}^Q$ ist, das eine lokale relative Strömungsgeschwindigkeit (126) an unterschiedlichen Positionen über den freien Querschnitt Q des Strömungskanals (94) in dem Blutgefäß-Modell ( $M_B^Q$ ) in Relation zu einer Referenz-Strömungsgeschwindigkeit ($v_R$) beschreibt.

7. Computerimplementiertes Verfahren nach Anspruch 6, **dadurch gekennzeichnet, dass** der durch den Strömungskanal (94) in dem Blutgefäß-Modell ( $M_B^Q$ ) geführte Fluidstrom berechnet wird, indem aus dem relativen Fluidströmungs-Modell $M_{Fr}^Q$ zu der Referenz-Strömungsgeschwindigkeit ($v_R$) sowie dem Fluoreszenzlicht-Modell $M_L^Q$ ein Korrekturfaktor $k\_v_R$ bestimmt wird und indem aus der Länge (L) des Abschnitts ($90_1$, $90_2$, $90_3$, ...) des Blutgefäßes (88) sowie aus der charakteristischen Transitzeit ($\tau$) für das Fluorophor in dem Abschnitt ($90_1$, $90_2$, $90_3$, ...) des Blutgefäßes (88) eine Fluorophor-Ausbreitungsgeschwindigkeit bestimmt wird, die mittels des Korrekturfaktors $k\_v_R$ auf einen der Referenz-Strömungsgeschwindigkeit ($v_R$) entsprechenden Wert korrigiert wird.

8. Computerimplementiertes Verfahren nach Anspruch 7, **dadurch gekennzeichnet, dass** der Korrekturfaktor $k\_v_R$ als Inverse des Erwartungswerts der relativen Strömungsgeschwindigkeiten in dem relativen Fluidströmungs-Modell $M_{Fr}^Q$ in Abhängigkeit von der durch das Fluoreszenzlicht-Modell $M_L^Q$ beschriebenen räumlichen Wahrscheinlichkeitsdichte für die Intensität des remittierten Lichts an unterschiedlichen Positionen über den freien Querschnitt (Q) des Strömungskanals in dem Blutgefäß-Modell ( $M_B^Q$ ) nach der folgenden Vorschrift bestimmt wird:

$$k\_v_R = \frac{1}{\int_Q M_{Fr}^Q(x)\, M_L^Q(x)dx}.$$

9. Computerimplementiertes Verfahren nach einem der Ansprüche 1 bis 8, **dadurch gekennzeichnet, dass** die Länge (L) des Abschnitts ($90_1$, $90_2$, $90_3$, ...) des Blutgefäßes (88) und/oder der Durchmesser (D) des Abschnitts ($90_1$, $90_2$, $90_3$, ...) des Blutgefäßes (88) anhand einer Mittellinie (98) des Abschnitts ($90_1$, $90_2$, $90_3$, ...) des Blutgefäßes (88) in wenigstens einem der bereitgestellten Bilder ($80_1$, $80_2$, $80_3$, $80_4$, ...) bestimmt wird.

10. Computerimplementiertes Verfahren nach Anspruch 9, **dadurch gekennzeichnet, dass** die Mittellinie (98) des Abschnitts ($90_1$, $90_2$, $90_3$, ...) des Blutgefäßes (88) bestimmt wird,

indem Pixel auf der Mittellinie (98) des Abschnitts ($90_1$, $90_2$, $90_3$, ...) des Blutgefäßes (88) durch Verarbeiten der bereitgestellten Bilder ($80_1$, $80_2$, $80_3$, $80_4$, ...) ermittelt werden,
indem aus den Pixeln der Mittellinie (98) ein Polygonzug (104) bestimmt wird,
indem die Länge des Polygonzugs (104) durch das Anpassen von Verbindungsstrukturen (110, 112) der Pixel entlang der Mittellinie (98) auf Basis ihrer Pixelnachbarschaften (108) minimiert wird,
und indem an den minimierten Polygonzug (104) kontinuierliche Funktionen (106) gefittet werden.

11. Computerimplementiertes Verfahren nach einem der Ansprüche 1 bis 10, **dadurch gekennzeichnet,**

**dass** das Fluidströmungs-Modell $M_F^Q$ und das Fluoreszenzlicht-Modell $M_L^Q$ einen lokalen Ausschnitt ($A_1$, $A_2$) des Blutgefäß-Modells $(M_B^Q)$ beschreiben, sodass das Urbild des Fluidströmungs-Modells $M_F^Q$ und des Fluoreszenzlicht-Modells $M_L^Q$ einen Teilbereich des freien Querschnitts Q des Blutgefäß-Modells $(M_B^Q)$ darstellt;
und/oder
**dass** die Länge (L), der Durchmesser (D), eine Mittellinie (98) des Abschnitts (90) des Blutgefäßes (88), das Blutgefäß-Modell $(M_B^Q)$, das Fluidströmungs-Modell $M_F^Q$ und/oder das Fluoreszenzlicht-Modell $M_L^Q$ aus einem anhand eines Kriteriums in Bezug auf die Intensität I(x) als Maß für die Bildhelligkeit der Bildpunkte des ausgewählten Bildes (164) ermittelt werden,
und/oder
**dass** die Transitzeit ($\tau$) aus dem Versatz einer Zeitentwicklung (160, 162) der Bildhelligkeit an wenigstens zwei unterschiedlichen Ausschnitten ($A_1$, $A_2$) des Abschnitts ($90_1$, $90_2$, $90_3$, ...) des Blutgefäßes (88) durch Verarbeiten der bereitgestellten Bilder ($80_1$, $80_2$, $80_3$, $80_4$, ...) ermittelt wird und dass an die Zeitentwicklung (160, 162) der Bildhelligkeit an den unterschiedlichen Ausschnitten ($A_1$, $A_2$) des Abschnitts ($90_1$, $90_2$, 90s, ...) des Blutgefäßes (88) jeweils eine kontinuierliche Funktion (106) gefittet wird;
und/oder
**dass** zu dem berechneten Blutvolumenfluss ($I_{Bl}$) in dem Abschnitt ($90_1$, $90_2$, $90_3$, ...) des Blutgefäßes (88) ein Vertrauensintervall auf Basis des Durchmessers (D) und/oder der Länge (L) des Abschnitts ($90_1$, $90_2$, $90_3$, ...) des Blutgefäßes (88) und/oder der Transitzeit ($\tau$) und/oder eines Korrekturfaktors ($k\_v_R$) und/oder des Blutgefäß-Modells ($M_B^Q$) und/oder des Fluidströmungs-Modells $M_F^Q$ und/oder des Fluoreszenzlicht-Modells $M_L^Q$ und/oder der Form einer Mittellinie (98) des Abschnitts ($90_1$, $90_2$, $90_3$, ...) des Blutgefäßes (88) anhand von Fehler-Simulationen bestimmt wird;
und/oder
**dass** der Abschnitt (90) des Blutgefäßes (88) durch Verarbeiten der bereitgestellten Bilder ($80_1$, $80_2$, $80_3$, $80_4$,...) mit einem Bildsegmentierungsverfahren bestimmt wird.

12. Computerimplementiertes Verfahren nach einem der Ansprüche 1 bis 11, **dadurch gekennzeichnet, dass** die Breite des Strömungskanals (94) sowie die Wandstärke (d) der Wand (95) des Blutgefäß-Modells ($M_B^Q$) anhand eines Kriteriums in Bezug auf eine Kurve (114) des Intensitätsverlaufs orthogonal zu einer Mittellinie (98) des Abschnitts (90) des Blutgefäßes (88) in einem oder mehreren der bereitgestellten Bilder ($80_1$, $80_2$, $80_3$, $80_4$,...) ermittelt werden.

13. Computerimplementiertes Verfahren nach Anspruch 12, **dadurch gekennzeichnet, dass** das Kriterium in Bezug auf die Kurve (114) des Intensitätsverlaufs ein Minimum der Krümmung der Kurve (114) des Intensitätsverlaufs orthogonal zu einer Mittellinie (98) des Abschnitts ($90_1$, $90_2$, $90_3$, ...) des Blutgefäßes (88) berücksichtigt.

14. Computerimplementiertes Verfahren (10) zur Bestimmung des Blutvolumenflusses ($I_B$) durch ein Blutgefäß (88) in einem Operationsbereich (36) anhand eines Fluorophors, bei dem das Blutgefäß (88) in mehrere Abschnitte ($90_1$, $90_2$, $90_3$, ...) zerlegt und der Blutvolumenfluss ($I_{Bi}$) in den Abschnitten ($90_1$, $90_2$, $90_3$, ...) jeweils nach einem der Ansprüche 1 bis 13 mit der Maßgabe bestimmt wird, dass an einer Verzweigung (89) des Blutgefäßes (88) die Summe der Blutvolumenflüsse ($I_{Bi}$) zu der Verzweigung (89) der Summe der Blutvolumenflüsse ($I_{Bi}$) von der Verzweigung (89) entspricht.

15. Computerprogramm mit Programmcode zur Durchführung aller Verfahrensschritte, die in einem der Ansprüche 1 bis 14 angegeben sind, wenn das Computerprogramm in eine Rechnereinheit (72) geladen und/oder in einer Rechnereinheit (72) durchgeführt wird.

16. Operationssystem zur Bestimmung des Blutvolumenflusses ($I_{BI}$) durch einen Abschnitt ($90_1$, $90_2$, $90_3$, ...) eines Blutgefäßes (88) in einem Operationsbereich (36) anhand eines Fluorophors,

mit einer Beleuchtungseinrichtung (42) zur Bereitstellung von Fluoreszenzanregungslicht für den Operationsbereich (36);
mit einer Bilderfassungseinrichtung (64) zur Bereitstellung einer Mehrzahl von Bildern ($80_1$, $80_2$, $80_3$, $80_4$,...), die auf Licht mit innerhalb eines Fluoreszenzspektrums des Fluorophors liegenden Wellenlängen basieren und die den Abschnitt (90) des Blutgefäßes (88) zu unterschiedlichen Zeitpunkten ($t_1$, $t_2$, $t_3$, $t_4$,...) zeigen;
und mit einer Rechnereinheit (72), die ein Computerprogramm nach Anspruch 15 enthält.

## Claims

1. Computer-implemented method (10) for determining the blood volume flow ($I_{BI}$) through a portion ($90_1$, $90_2$, $90_3$, ...) of a blood vessel (88) in an operating region (36) using a fluorophore,

in which a plurality of images ($80_1$, $80_2$, $80_3$, $80_4$,...) are provided, which are based on fluorescent light in the form of light having wavelengths lying within a fluorescence spectrum of the fluorophore, and which show the portion ($90_2$, $90_2$, $90_3$, ...) of the blood vessel (88) at different recording times (t1, t2, t3, t4,...),
in which, by processing the provided images ($80_1$, $80_2$, $80_3$, $80_4$, ...), a diameter (D) and a length (L) of the portion (90) of the blood vessel (88) and a time interval for a propagation of the fluorophore through the portion ($90_2$, $90_2$, $90_3$, ...) of the blood vessel (88) are determined, which time interval describes a characteristic transit time ($\tau$) for the fluorophore in the portion ($90_2$, $90_2$, $90_3$, ...) of the blood vessel (88),

in which a blood vessel model ( $M_B^Q$ ), which describes the portion ($90_2$, $90_2$, $90_3$, ...) of the blood vessel (88) as a flow channel (94) having a length (L), having a wall (95) with a wall thickness (d), and having a free cross section Q, on at least one of the provided images ($80_1$, $80_2$, $80_3$, $80_4$,...) is adapted by means of image processing;

in which a fluid flow model $M_F^Q$ for the adapted blood vessel model $(M_B^Q)$ is provided, which fluid flow model describes a local flow velocity (122) at different positions over the free cross section Q of the flow channel (94) in the adapted blood vessel model $(M_B^Q)$;
**characterized in that**

a fluorescent light model $M_L^Q$ is provided, which describes a spatial probability density for the intensity of the diffusely reflected light at different positions over the free cross section Q of the flow channel (94) in the adapted blood vessel model $(M_B^Q)$, which light is emitted by a fluid, which is mixed with fluorophore and flows through the free cross section Q of the flow channel (94) in the adapted blood vessel model $(M_B^Q)$, when said fluid is irradiated with fluorescence excitation light; and the blood volume flow ($I_{Bl}$) is determined as a fluid flow guided through the flow channel (94) in the adapted blood vessel model $(M_B^Q)$, which fluid flow is calculated from the length (L) and the diameter (D) of the portion ($90_2$, $90_2$, $90_3$, ...) of the blood vessel (88) and from the characteristic transit time ($\tau$) for the fluorophore in the portion ($90_2$, $90_2$, $90_3$, ...) of the blood vessel (88), using the fluid flow model $M_F^Q$ provided and the fluorescent light model $M_L^Q$ provided.

2. Computer-implemented method according to Claim 1, **characterized in that** the plurality of images (801, 802, 803, 804, ...) are acquired using an image acquisition device (64), wherein, during the determination of the blood volume flow ($I_{Bl}$), parameters of the image acquisition device (64), of the blood vessel model $(M_B^Q)$, of the fluid flow model $M_F^Q$ and of the fluorescent light model $M_L^Q$ are not changed.

3. Computer-implemented method according to Claim 1 or 2, **characterized in that** the blood vessel model $(M_B^Q)$ is a hollow cylinder with length (L), diameter (D) and wall thickness (d),
and/or

**in that** the fluid flow model $M_F^Q$ describes a laminar fluid flow through the flow channel (94) of the blood vessel model $(M_B^Q)$.

4. Computer-implemented method according to any of Claims 1 to 3, **characterized in that** the fluorescent light model $M_L^Q$ is based on a simulation of an irradiation of the blood vessel model $(M_B^Q)$ with fluorescence excitation light, in which photons (127) are assumed to be particles scattered at scattering centres, the scattering centres in the flow channel (94) and in the wall (95) of the blood vessel model $(M_B^Q)$ each having a characteristic scattering centre distribution.

5. Computer-implemented method according to Claim 4, **characterized in that** the archetype of the fluorescent light model $M_L^Q$ corresponds to a chord of the free cross section Q of the blood vessel model $(M_B^Q)$ which represents the penetration depth of the photons (127) into the blood vessel model ($M_B^Q$) upon irradiation with fluorescence excitation light, and **in that** the fluorescent light model $M_L^Q$ represents the penetration depth x on the proportion of the photons (127) diffusely reflected from the blood vessel model $(M_B^Q)$, the maximum penetration depth of which photons in the blood vessel model $(M_B^Q)$ corresponds to the value x during the simulation.

6. Computer-implemented method according to any of Claims 1 to 5, **characterized in that** the fluid flow model $M_F^Q$ is a relative fluid flow model $M_{Fr}^Q$ which describes a local relative flow velocity (126) at different positions over the free cross section Q of the flow channel (94) in the blood vessel model $(M_B^Q)$ in relation to a reference flow velocity $(v_R)$.

7. Computer-implemented method according to Claim 6, **characterized in that** the fluid flow guided through the flow channel (94) in the blood vessel model $(M_B^Q)$ is calculated by using the relative fluid flow model $M_{Fr}^Q$ to the reference flow velocity $(v_R)$ and the fluorescent light model $M_L^Q$ to determine a correction factor $k\_v_R$, and by using the length (L) of the portion ($90_1$, $90_2$, $90_3$, ...) of the blood vessel (88) and the characteristic transit time $(\tau)$ for the fluorophore in the portion ($90_1$, $90_2$, $90_3$, ...) of the blood vessel (88) to determine a fluorophore propagation speed which, by means of the correction factor $k\_v_R$, is corrected to a value corresponding to the reference flow velocity $(v_R)$.

8. Computer-implemented method according to Claim 7, **characterized in that** the correction factor $k\_v_R$, as the inverse of the expected value of the relative flow velocities in the relative fluid flow model $M_{Fr}^Q$, dependent on the spatial probability density described by the fluorescent light model $M_L^Q$ for the intensity of the diffusely reflected light at different positions over the free cross section (Q) of the flow channel in the blood vessel model $(M_B^Q)$, is determined according to the following rule:

$$k\_v_R = \frac{1}{\int_Q M_{Fr}^Q(x)\, M_L^Q(x)\, dx}.$$

9. Computer-implemented method according to any of Claims 1 to 8, **characterized in that** the length (L) of the portion ($90_2$, $90_2$, $90_3$, ...) of the blood vessel (88) and/or the diameter (D) of the portion ($90_1$, $90_2$, $90_3$, ...) of the blood vessel (88) is determined, on the basis of a centre line (98) of the portion ($90_2$, $90_2$, $90_3$, ...) of the blood vessel (88), in at least one of the provided images ($80_1$, $80_2$, $80_3$, $80_4$,...).

10. Computer-implemented method according to Claim 9, **characterized in that** the centre line (98) of the portion ($90_2$, $90_2$, $90_3$, ...) of the blood vessel (88) is ascertained by determining pixels on the centre line (98) of the portion ($90_2$, $90_2$, $90_3$, ...) of the blood vessel (88) by processing the provided images ($80_1$, $80_2$, $80_3$, $80_4$, ,...),

by determining a polygonal chain (104) from the pixels of the centre line (98),
by minimizing the length of the polygonal chain (104) by adapting connection structures (110, 112) of the pixels along the centre line (98) on the basis of their pixel neighbourhoods (108),
and by fitting continuous functions (106) to the minimized polygonal chain (104).

11. Computer-implemented method according to any of Claims 1 to 10, **characterized in that**

the fluid flow model $M_F^Q$ and the fluorescent light model $M_L^Q$ describe a local sector ($A_1$, $A_2$) of the blood vessel model $(M_B^Q)$, such that the archetype of the fluid flow model $M_F^Q$ and of the fluorescent light model $M_L^Q$ represents a partial region of the free cross section Q of the blood vessel model $(M_B^Q)$;
and/or
**in that** the length (L), the diameter (D), a centre line (98) of the portion (90) of the blood vessel (88), the blood

vessel model $(M_B^Q)$, the fluid flow model $M_F^Q$ and/or the fluorescent light model $M_L^Q$ are determined using a criterion relating to the intensity **I(x)** as a measure of the image brightness of the pixels of the selected image (164),

and/or

**in that** the transit time ($\tau$) is determined from the offset of a time evolution (160, 162) of the image brightness at at least two different sectors ($A_1$, $A_2$) of the portion ($90_2$, $90_2$, $90_3$, ...) of the blood vessel (88) by processing the provided images ($80_1$, $80_2$, $80_3$, $80_4$,...), and **in that** a continuous function (106) is in each case fitted to the time evolution (160, 162) of the image brightness at the different sectors ($A_1$, $A_2$) of the portion ($90_1$, $90_2$, $90_3$, ...) of the blood vessel (88);

and/or

**in that**, for the calculated blood volume flow ($I_{BI}$) in the portion ($90_2$, $90_2$, $90_3$, ...) of the blood vessel (88), a confidence interval based on the diameter (D) and/or the length (L) of the portion ($90_2$, $90_2$, $90_3$, ...) of the blood vessel (88) and/or the transit time ($\tau$) and/or a correction factor ($k\_v_R$)) and/or the blood vessel model $(M_B^Q)$ and/or the fluid flow model $M_F^Q$ and/or the fluorescent light model $M_L^Q$ and/or the shape of a centre line (98) of the portion ($90_2$, $90_2$, $90_3$, ...) of the blood vessel (88) is determined on the basis of error simulations;

and/or

**in that** the portion (90) of the blood vessel (88) is determined by processing the provided images ($80_1$, $80_2$, $80_3$, $80_4$,...) using an image segmentation method.

12. Computer-implemented method according to any of Claims 1 to 11, **characterized in that** the width of the flow channel (94) and the wall thickness (d) of the wall (95) of the blood vessel model $(M_B^Q)$ are determined, on the basis of a criterion relating to a curve (114) of the intensity profile orthogonal to a centre line (98) of the portion (90) of the blood vessel (88), in one or more of the provided images ($80_1$, $80_2$, $80_3$, $80_4$,...).

13. Computer-implemented method according to Claim 12, **characterized in that** the criterion relating to the curve (114) of the intensity profile takes into account a minimum of the curvature of the curve (114) of the intensity profile orthogonal to a centre line (98) of the portion ($90_2$, $90_2$, $90_3$, ...) of the blood vessel (88) .

14. Computer-implemented method (10) for determining the blood volume flow ($I_B$) through a blood vessel (88) in an operating region (36) using a fluorophore, in which the blood vessel (88) is divided into several portions ($90_1$, $90_2$, $90_3$, ...) and the blood volume flow ($I_{Bi}$) in the portions ($90_2$, $90_2$, $90_3$, ...) is determined according to any of Claims 1 to 13, with the proviso that, at a branch (89) of the blood vessel (88), the sum of the blood volume flows ($I_{Bi}$) to the branch (89) corresponds to the sum of the blood volume flows ($I_{Bi}$) from the branch (89).

15. Computer program having a program code for carrying out all method steps, specified in any of Claims 1 to 14, when the computer program is loaded on a computer unit (72) and/or executed on a computer unit (72).

16. Surgical system for determining the blood volume flow ($I_{BI}$) through a portion ($90_1$, $90_2$, $90_3$, ...) of a blood vessel (88) in an operating region (36) using a fluorophore,

with an illumination device (42) for providing fluorescence excitation light for the operating region (36);
with an image acquisition device (64) for providing a plurality of images ($80_1$, $80_2$, $80_3$, $80_4$,...), which are based on light having wavelengths lying within a fluorescence spectrum of the fluorophore, and which show the portion (90) of the blood vessel (88) at different times ($t_1$, $t_2$, $t_3$, $t_4$,...);
and with a computer unit (72) containing a computer program according to Claim 15.

**Revendications**

1. Procédé mis en oeuvre par ordinateur (10) pour déterminer le débit volumique sanguin ($I_{BI}$) à travers une partie ($90_2$, $90_2$, $90_3$, ...) d'un vaisseau sanguin (88) dans une zone chirurgicale (36) à l'aide d'un fluorophore,

dans lequel une pluralité d'images ($80_1$, $80_2$, $80_3$, $80_4$, ...) est fournie qui est basée sur une lumière fluorescente sous la forme d'une lumière ayant des longueurs d'onde comprises dans un spectre de fluorescence du fluorophore et qui montre la partie ($90_1$, $90_2$, $90_3$, ...) du vaisseau sanguin (88) à différents temps d'enregistrement ($t_1$, $t_2$, $t_3$, $t_4$, ...),

dans lequel le traitement des images fournies ($80_1$, $80_2$, $80_3$, $80_4$, ...) permet d'établir un diamètre (D) et une longueur (L) de la partie (90) du vaisseau sanguin (88) ainsi qu'un intervalle de temps pour une propagation du fluorophore à travers la partie ($90_1$, $90_2$, $90_3$, ...) du vaisseau sanguin (88) qui décrit un temps de transit caractéristique (T) pour le fluorophore dans la partie ($90_2$, $90_2$, $90_3$, ...) du vaisseau sanguin (88),

dans lequel un modèle de vaisseau sanguin $\left(M_B^Q\right)$, qui décrit la partie ($90_1$, $90_2$, $90_3$, ...) du vaisseau sanguin (88) comme un canal d'écoulement (94) ayant une longueur (L), une paroi (95) d'une épaisseur de paroi (d) et une section transversale libre Q, est adapté à au moins l'une des images fournies ($80_1$, $80_2$, $80_3$, $80_4$, ...) au moyen d'un traitement d'image ;

dans lequel un modèle d'écoulement de fluide $M_F^Q$ est fourni pour le modèle de vaisseau sanguin adapté $\left(M_B^Q\right)$, qui décrit une vitesse d'écoulement locale (122) à différentes positions sur la section transversale libre Q du canal d'écoulement (94) dans le modèle de vaisseau sanguin $\left(M_B^Q\right)$ adapté ;

**caractérisé en ce que**

un modèle de lumière fluorescente $M_L^Q$ est fourni qui décrit une densité de probabilité spatiale pour l'intensité de la lumière renvoyée à différentes positions sur la section transversale libre Q du canal d'écoulement (94) dans le modèle de vaisseau sanguin adapté $\left(M_B^Q\right)$, ladite lumière étant émise par un fluide mélangé au fluorophore, passant par la section transversale libre Q du canal d'écoulement (94) dans le modèle de vaisseau sanguin adapté $\left(M_B^Q\right)$ en cas d'irradiation avec une lumière d'excitation en fluorescence ; et

le débit volumique sanguin ($I_{Bl}$) est déterminé comme un écoulement de fluide guidé à travers le canal d'écoulement (94) dans le modèle de vaisseau sanguin adapté $\left(M_B^Q\right)$, ledit écoulement étant calculé à l'aide du modèle d'écoulement de fluide fourni $M_F^Q$ et du modèle de lumière fluorescente fourni $M_L^Q$ à partir de la longueur (L) et du diamètre (D) de la partie ($90_1$, $90_2$, $90_3$, ...) du vaisseau sanguin (88) ainsi qu'à partir du temps de transit caractéristique (T) pour le fluorophore dans la partie ($90_2$, $90_2$, $90_3$, ...) du vaisseau sanguin (88) .

2. Procédé mis en oeuvre par ordinateur selon la revendication 1, **caractérisé en ce que** la pluralité d'images ($80_1$, $80_2$, $80_3$, $80_4$, ...) est acquise par un dispositif d'acquisition d'image (64), dans lequel pendant la détermination du débit volumique sanguin ($I_{Bl}$) des paramètres du dispositif d'acquisition d'image (64) du modèle de vaisseau sanguin $\left(M_B^Q\right)$, du modèle d'écoulement de fluide $M_F^Q$ et du modèle de lumière fluorescente $M_L^Q$ ne sont pas modifiés.

3. Procédé mis en oeuvre par ordinateur selon la revendication 1 ou 2, **caractérisé en ce que** le modèle de vaisseau sanguin $\left(M_B^Q\right)$ est un cylindre creux ayant une longueur (L), un diamètre (D) et une épaisseur de paroi (d), et/ou

**en ce que** le modèle d'écoulement de fluide $M_F^Q$ décrit un écoulement de fluide laminaire à travers le canal d'écoulement (94) du modèle de vaisseau sanguin $\left(M_B^Q\right)$ .

4. Procédé mis en oeuvre par ordinateur selon l'une quelconque des revendications 1 à 3, **caractérisé en ce que** le modèle de lumière fluorescente $M_L^Q$ est basé sur une simulation d'une irradiation du modèle de vaisseau sanguin $\left(M_B^Q\right)$ avec une lumière d'excitation en fluorescence, dans laquelle des photons (127) sont supposés être des particules dispersées au niveau de centres de dispersion, dans lequel les centres de dispersion présentent dans le canal d'écoulement (94) et dans la paroi (95) du modèle de vaisseau sanguin $\left(M_B^Q\right)$ une distribution de centres de dispersion caractéristique respectivement.

5. Procédé mis en oeuvre par ordinateur selon la revendication 4, **caractérisé en ce que** l'image originale du modèle de lumière fluorescentes $M_L^Q$ correspond à une corde de la section transversale libre Q du modèle de vaisseau sanguin $\left(M_B^Q\right)$ qui représente la profondeur de pénétration des photons (127) dans le modèle de vaisseau sanguin $\left(M_B^Q\right)$ en cas d'irradiation avec une lumière d'excitation en fluorescence, et **en ce que** le modèle de lumière fluorescente $M_L^Q$ mappe la profondeur de pénétration x sur la fraction des photons (127) renvoyés à partir du modèle de vaisseau sanguin $\left(M_B^Q\right)$ dont la profondeur de pénétration maximale dans le modèle de vaisseau sanguin $\left(M_B^Q\right)$ correspond à la valeur x pendant la simulation.

6. Procédé mis en oeuvre par ordinateur selon l'une quelconque des revendications 1 à 5, **caractérisé en ce que** le modèle d'écoulement de fluide $M_F^Q$ est un modèle d'écoulement de fluide relatif $\left(M_{Fr}^Q\right)$ qui décrit une vitesse d'écoulement relative locale (126) à différentes positions sur la section transversale libre Q du canal d'écoulement (94) dans le modèle de vaisseau sanguin $\left(M_B^Q\right)$ par rapport à une vitesse d'écoulement de référence ($v_R$).

7. Procédé mis en oeuvre par ordinateur selon la revendication 6, **caractérisé en ce que** le flux de fluide guidé à travers le canal d'écoulement (94) dans le modèle de vaisseau sanguin $\left(M_B^Q\right)$ est calculé **en ce qu'**un facteur de correction k_$v_R$ est déterminé à partir du modèle d'écoulement de fluide relatif $M_{Fr}^Q$ pour la vitesse d'écoulement de référence ($v_R$) ainsi qu'à partir du modèle de lumière fluorescente $M_L^Q$, et **en ce qu'**une vitesse de propagation de fluorophore est déterminée à partir de la longueur (L) de la partie ($90_1$, $90_2$, $90_3$, ...) du vaisseau sanguin (88) ainsi qu'à partir du temps de transit caractéristique (T) pour le fluorophore dans la partie ($90_1$, $90_2$, $90_3$, ...) du vaisseau sanguin (88), ladite vitesse étant corrigée au moyen du facteur de correction k_$v_R$ à une valeur correspondant à la vitesse d'écoulement de référence ($v_R$).

8. Procédé mis en oeuvre par ordinateur selon la revendication 7, **caractérisé en ce que** le facteur de correction k_$v_R$ est déterminé comme l'inverse de la valeur attendue des vitesses d'écoulement relatives dans le modèle d'écoulement de fluide relatif $M_{Fr}^Q$ en fonction de la densité de probabilité spatiale décrite par le modèle de lumière fluorescente $M_L^Q$ pour l'intensité de la lumière renvoyée à différentes positions sur la section transversale libre (Q) du canal d'écoulement dans le modèle de vaisseau sanguin $\left(M_B^Q\right)$ selon la règle suivante :

$$k\_\vartheta_R = \frac{1}{\int_Q M_{Fr}^Q(x)M_L^Q(x)dx}$$

9. Procédé mis en oeuvre par ordinateur selon l'une quelconque des revendications 1 à 8, **caractérisé en ce que** la longueur (L) de la partie ($90_1$, $90_2$, $90_3$, ...) du vaisseau sanguin (88) et/ou le diamètre (D) de la partie ($90_2$, $90_2$, $90_3$, ...) du vaisseau sanguin (88) sont déterminés à l'aide d'une ligne médiane (98) de la partie ($90_1$, $90_2$, $90_3$, ...) du vaisseau sanguin (88) sur au moins l'une des images fournies ($80_1$, $80_2$, $80_3$, $80_4$, ...) .

10. Procédé mis en oeuvre par ordinateur selon la revendication 9, **caractérisé en ce que** la ligne médiane (98) de la partie ($90_2$, $90_2$, $90_3$, ...) du vaisseau sanguin (88) est déterminée,

en ce que des pixels sur la ligne médiane (98) de la partie ($90_2$, $90_2$, $90_3$, ...) du vaisseau sanguin (88) sont établis par le traitement des images fournies ($80_1$, $80_2$, $80_3$, $80_4$, ...),
**en ce qu'**un tracé polygonal (104) est déterminé à partir des pixels de la ligne médiane (98),
**en ce que** la longueur du tracé polygonal (104) est minimisée par l'adaptation de structures de connexion (110, 112) des pixels le long de la ligne médiane (98) sur la base de leur voisinage de pixels (108),
et **en ce que** des fonctions continues (106) sont ajustées sur le tracé polygonal (104) minimisé.

11. Procédé mis en oeuvre par ordinateur selon l'une quelconque des revendications 1 à 10, **caractérisé en ce que**

le modèle d'écoulement de fluide $M_F^Q$ et le modèle de lumière fluorescente $M_L^Q$ décrivent une section locale ($A_1$, $A_2$) du modèle de vaisseau sanguin $\left(M_B^Q\right)$ de sorte que l'image originale du modèle d'écoulement de fluide $M_F^Q$ et du modèle de lumière fluorescente $M_L^Q$ représente une zone partielle de la section transversale libre Q du modèle de vaisseau sanguin $\left(M_B^Q\right)$ ;
et/ou
**en ce que** la longueur (L), le diamètre (D), une ligne médiane (98) de la partie (90) du vaisseau sanguin (88), le modèle de vaisseau sanguin $\left(M_B^Q\right)$, le modèle d'écoulement de fluide $M_F^Q$ et/ou le modèle de lumière fluorescente $M_L^Q$ sont établis à l'aide d'un critère concernant l'intensité I(x) comme une mesure de la luminosité d'image des pixels de l'image sélectionnée (164),
et/ou
**en ce que** le temps de transit (T) est établi à partir du décalage d'une évolution dans le temps (160, 162) de la luminosité d'image sur au moins deux sections différentes ($A_1$, $A_2$) de la partie ($90_1$, $90_2$, $90_3$, ...) du vaisseau sanguin (88) par le traitement des images fournies ($80_1$, $80_2$, $80_3$, $80_4$, ...), et **en ce qu'**une fonction continue (106) est respectivement ajustée sur l'évolution dans le temps (160, 162) de la luminosité d'image aux différentes sections ($A_1$, $A_2$) de la partie ($90_1$, $90_2$, $90_3$, ...) du vaisseau sanguin (88) ;
et/ou
**en ce qu'**un intervalle de confiance est déterminé pour le débit volumique sanguin ($I_{BI}$) dans la partie ($90_1$, $90_2$, $90_3$, ...) du vaisseau sanguin (88) sur la base du diamètre (D) et/ou de la longueur (L) de la partie ($90_1$, $90_2$, $90_3$, ...) du vaisseau sanguin (88) et/ou du temps de transit (T) et/ou d'un facteur de correction ($k\_v_R$) et/ou du modèle de vaisseau sanguin $\left(M_B^Q\right)$ et/ou du modèle d'écoulement de fluide $M_F^Q$ et/ou du modèle de lumière fluorescentes $M_L^Q$ et/ou de la forme d'une ligne médiane (98) de la partie ($90_2$, $90_2$, $90_3$, ...) du vaisseau sanguin (88) à l'aide de simulations d'erreurs ;
et/ou
**en ce que** la partie (90) du vaisseau sanguin (88) est déterminée par le traitement des images fournies ($80_1$, $80_2$, $80_3$, $80_4$, ...) par un procédé de segmentation d'image.

12. Procédé mis en oeuvre par ordinateur selon l'une quelconque des revendications 1 à 11, **caractérisé en ce que** la largeur du canal d'écoulement (94) ainsi que l'épaisseur de paroi (d) de la paroi (95) du modèle de vaisseau sanguin $\left(M_B^Q\right)$ sont établies à l'aide d'un critère concernant une courbe (114) du tracé d'intensité, orthogonalement à une ligne médiane (98) de la partie (90) du vaisseau sanguin (88) sur une ou plusieurs des images fournies ($80_1$, $80_2$, $80_3$, $80_4$, ...) .

**13.** Procédé mis en oeuvre par ordinateur selon la revendication 12, **caractérisé en ce que** le critère concernant la courbe (114) du tracé d'intensité tient compte d'un minimum de la courbure de la courbe (114) du tracé d'intensité, orthogonalement à une ligne médiane (98) de la partie ($90_2$, $90_2$, $90_3$, ...) du vaisseau sanguin (88) .

**14.** Procédé mis en oeuvre par ordinateur (10) permettant de déterminer le débit volumique sanguin ($I_{BI}$) à travers un vaisseau sanguin (88) dans une zone chirurgicale (36) à l'aide d'un fluorophore, dans lequel le vaisseau sanguin (88) est décomposé en plusieurs parties ($90_1$, $90_2$, $90_3$, ...) et le débit volumique sanguin ($I_{BI}$) dans les parties ($90_1$, $90_2$, $90_3$, ...) est déterminé respectivement selon l'une quelconque des revendications 1 à 13 à condition qu'au niveau d'une ramification (89) du vaisseau sanguin (88), la somme des débits volumiques sanguins ($I_{BI}$) vers la ramification (89) corresponde à la somme des débits volumiques sanguins ($I_{BI}$) de la ramification (89).

**15.** Programme informatique comprenant du code programme pour exécuter toutes les étapes de procédé qui sont indiquées dans l'une quelconque des revendications 1 à 14 si le programme informatique est chargé sur une unité de calcul (72) et/ou est exécuté sur une unité de calcul (72) .

**16.** Système d'exploitation permettant de déterminer le débit volumique sanguin ($I_{BI}$) à travers une partie ($90_1$, $90_2$, $90_3$, ...) d'un vaisseau sanguin (88) dans une zone chirurgicale (36) à l'aide d'un fluorophore, comprenant

un dispositif d'éclairage (42) pour fournir une lumière d'excitation en fluorescence pour la zone chirurgicale (36) ;
un dispositif d'acquisition d'image (64) pour fournir une pluralité d'images ($80_1$, $80_2$, $80_3$, $80_4$, ...) qui sont basées sur la lumière ayant des longueurs d'onde comprises dans un spectre de fluorescence du fluorophore et qui montrent la partie (90) du vaisseau sanguin (88) à différents temps ($t_1$, $t_2$, $t_3$, $t_4$, ...) ;
et une unité de calcul (72) qui comporte un programme informatique selon la revendication 15.

Fig.1

Fig.2

Fig.3

Fig.4A

Fig.4B

Fig.6A

Fig.6B

Fig.7A

Fig.7B

Fig.7C

Fig.7D

EP 4 100 877 B1

Fig.8

Fig.9A

Fig.9B

Fig.10

Fig.11

Fig.12

## Fig.13A

## Fig.13B

## Fig.13C

## Fig.13D

$$M^Q_{F_\Gamma}(x)$$

$$M^Q_L(x)$$

EP 4 100 877 B1

Fig.14 A

Fig.14 B

EP 4 100 877 B1

Fig.15

EP 4 100 877 B1

Fig.16

EP 4 100 877 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 3047796 A1

**In der Beschreibung aufgeführte Nicht-Patentliteratur**

- **CLAUDIA WEICHELT et al.** Quantitative fluorescence angiography for neurosurgical interventions. *Biomed Tech,* 2013, vol. 58 (3), 269-279
- **TSUKIYAMA, A. ; MURAI, Y. ; MATANO, F. ; SHIROKANE, K. ; MORITA, A.** Optical effects on the surrounding structure during quantitative analysis using indocyanine green videoangiography: A phantom vessel study. *J. Biophotonics,* 2018, ISSN 1864-0648, 11
- **XU, J. ; SONG, S. ; LI, Y. ; WANG, R.** Complex-based OCT angiography algorithm recovers microvascular information superior to amplitude or phasebased algorithm in phase-stable systems. *Physics in medicine and biology,* 19. Dezember 2017, vol. 63, ISSN 1361-6560, 1
- **SAITO et al.** Quantitative Blood Flow Assessment by Multiparameter Analysis of Indocyanine Green Video Angiography. *World Neurosurgery,* 2018, vol. 116, 187-193

- **NOBUYUKI OTSU.** A threshold selection method from gray-level histograms. *IEEE Trans. Sys. Man. Cyber.,* 1979, vol. 9 (1), 62-66
- **P. GOLLAND ; W. GRIMSON.** Fixed Topology Skeletons. *International Conference on Computer Vision and Pattern Recognition (CVPR),* 2000
- **NOBUYUKI OTSU.** A threshold selection method from gray-level histograms. *IEEE Trans. Sys. Man. Cyber.,* 1979, vol. 9 (1), 882-886
- **A. NABER ; D. BERWANGER ; W. NAHM.** In Silico Modling of Blood Vessel Segmentations for Estimation of Discretization Error in Spatial Measurement and its Impact on Quantitative Fluorescence Angiography. *41st Annual International Conference of the IEEE Engineering in Medicine and Biology Society (EMBC),* 2019